# EUROPEAN PATENT SPECIFICATION

(11) **EP 1 135 463 B1**
(45) Date of publication and mention of the grant of the patent: **09.01.2008**
(21) Application number: 99960304.6
(22) Date of filing: 12.11.1999
(51) Int. Cl.: C12N 5/06, C12N 5/08, A61L 27/38

(54) **LYMPHOID TISSUE-SPECIFIC CELL PRODUCTION FROM HEMATOPOIETIC PROGENITOR CELLS IN THREE-DIMENSIONAL DEVICES**
PRODUKTION LYMPHOIDER GEWEBSSPEZIFISCHER ZELLEN VON HÄMATOPOIETISCHEN VORLÄUFERZELLEN IN EINEM DREIDIMENSIONALEN SYSTEM
PRODUCTION DE CELLULES LYMPHOIDES SPECIFIQUES D'UN TISSU A PARTIR DE CELLULES SOUCHES HEMATOPOIETIQUES SE TROUVANT DANS DES DISPOSITIFS TRIDIMENSIONNELS

(30) Priority: 12.11.1998 US 107972 P
(43) Date of publication of application: 26.09.2001
(73) Proprietor: Cytomatrix, LLC, Massachusetts 01824 (US); THE GENERAL HOSPITAL CORPORATION, Boston, MA 02114 (US)
(72) Inventor: ROSENZWEIG, Michael, Boston, MA 02116 (US); PYKETT, Mark, J., Boxford, MA 01921 (US); SCADDEN, David T., Massachusetts General Hosp.East, Charlestown, MA 02129 (US); POZNANSKY, Mark C, Massachusetts General Hosp.East, Charlestown, MA 02129 (US)
(74) Representative: Jump, Timothy John Simon
(86) International application number: PCT/US1999/026795
(87) International publication number: WO 2000/027999

(56) References cited:
- EP-A- 0 241 578
- EP-A- 0 358 506
- WO-A-90/15877
- US-A- 5 443 950
- US-A- 5 580 781
- NAUGHTON G ET AL: "Three-dimensional bone marrow cell and tissue culture system" BIOTECHNOLOGY ADVANCES,GB,ELSEVIER PUBLISHING, BARKING, vol. 15, no. 2, 1 January 1997 (1997-01-01), page 401 XP004073886 ISSN: 0734-9750
- NAUGHTON B A ET AL: "A THREE-DIMENSIONAL CULTURE SYSTEM FOR THE GROWTH OF HEMA- TOPOIETIC CELLS" PROGRESS IN CLINICAL AND BIOLOGICAL RESEARCH,US,NEW YORK, NY, vol. 333, 1990, pages 435-445, XP000881141 ISSN: 0361-7742
- ROSENZWEIG M ET AL: "Enhanced maintenance and retroviral transduction of primitive hematopoietic progenitor cells using a novel three-dimensional culture system" GENE THERAPY,GB,MACMILLAN PRESS LTD., BASINGSTOKE, vol. 4, no. 9, 1 September 1997 (1997-09-01), pages 928-936, XP002091698 ISSN: 0969-7128
- BAGLEY J ET AL: "LONG-TERM THREE DIMENSIONAL HEMATOPOIETIC STEM CELL CULTURE" AMERICAN CHEMICAL SOCIETY. ABSTRACTS OF PAPER. AT THE NATIONAL MEETING,US,AMERICAN CHEMICAL SOCIETY, WASHINGTON, DC, vol. 216, no. 1/03, 1998, page BIOT129 XP000881132 ISSN: 0065-7727
- BAGLEY J ET AL: "EXTENDED CULTURE OF MULTIPOTENT HEMATOPOIETIC PROGENITORS WITHOUT CYTOKINE AUGMENTATION IN A NOVEL THREE-DIMENSIONAL DEVICE" EXPERIMENTAL HEMATOLOGY,US,NEW YORK, NY, vol. 27, no. 3, March 1999 (1999-03), pages 496-504, XP000881139 ISSN: 0301-472X

## Description

### Field of the Invention

The invention pertains to the co-culture of hematopoietic progenitor cells and lymphoreticular stromal cells in three-dimensional devices, resulting in unexpectedly high numbers of lymphoid tissue-specific cell progeny.

### Background of the Invention

A characteristic of the immune system is the specific recognition of antigens. This includes the ability to discriminate between self and non-self antigens and a memory-like potential that enables a fast and specific reaction to previously encountered antigens. The vertebrate immune system reacts to foreign antigens with a cascade of molecular and cellular events that ultimately results in the humoral and cell-mediated immune response.

The major pathway of the immune defense involving antigen-specific recognition commences with the trapping of the antigen by antigen presenting cells (APCs), such as dendritic cells or macrophages, and the subsequent migration of these cells to lymphoid organs (e.g., thymus). There, the APCs present antigen to subclasses of T cells classified as mature T helper cells. Upon specific recognition of the presented antigen, the mature T helper cells can be triggered to become activated T helper cells. The activated T helper cells regulate both the humoral immune response by inducing the differentiation of mature B cells to antibody producing plasma cells and the cell-mediated immune response by activation of mature cytotoxic T cells.

The thymus has been shown to be an obligatory factor in T cell differentiation of hematopoietic cells. Based upon the murine model, it is believed that the presence of a three dimensional organ is required, as *in vitro* models that do not include the thymus and a three dimensional structure fail to support T cell lymphopoiesis (Owen JJ, et al., Br Med Bull., 1989,45: 350-360). The process of differentiation, however, appears to begin prior to progenitor cells contacting the thymus.

Primitive hematopoietic progenitors in the fetal liver or bone marrow give rise to lineage committed cells, including progenitors committed to the T lymphoid lineage. These most immature cells are identified by the surface expression of CD34. T cell lineage committed cells express CD34, but no discrete expression of other epitopes found only on T cell progenitors has been described. Further, T lymphocyte differentiation normally occurs via a series of discrete developmental stages. Primitive progenitor cells which do not express lymphocyte specific cell surface markers (CD34+ CD3-CD4-CD8-) migrate to the thymus where they acquire, through a series of maturational events, the phenotype CD34-CD3 CD4+ CD8-. These cells then mature into double positive CD4+ CD8+ cells, most of which are CD3+, although CD3 expression is not universally detectable. These cells further undergo both positive and negative selection, and mature to develop into single positive T cells (CD4+ CD8-or CD4-CD8+). These cells ultimately migrate into the peripheral circulation as naive T cells.

T cell disorders and diseases represent major health problems. Recent progress has been made using gene therapy to treat conditions involving T lymphocytes, including AIDS. This has fostered increased interest in the development of laboratory techniques that allow *in vitro* evaluations of potential genetic therapies for these conditions.

The understanding of T cell differentiation has been hampered by the limited availability of technologies which permit *in vitro* T cell differentiation. To date, T cell differentiation studies have been largely confined to the SCID-hu mouse *in vivo* model. *In vitro* technologies have been based on thymic explant studies and primate thymic monolayers. In a recent advance, primate thymic stroma cultures have been shown to provide an expedient, although inefficient, system for examining T cell development, enabling *in vitro* T cell differentiation in a reproducible manner. However, the purity and number of T cells generated this way, as well as the relatively short half-life of the cultures, generally results in limited applicability to more advanced studies of T cell differentiation and function.

### Summary of the Invention

The invention, in one important part, involves improved methods for culturing hematopoietic progenitor cells that direct their development toward lymphoid tissue-specific lineages without the addition of exogenous growth factors wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells. Thus, one aspect of the invention is the culture of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, to generate progeny committed to a specific lineage. Another aspect is an improvement in the rate and the number of differentiated progeny that can be obtained from a sample of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

We describe herein a system that takes advantage of biocompatible, open-pore, three-dimensional matrices, and uses human and non-human lymphoreticular stromal cells to provide the appropriate conditions for the expansion and differentiation of human and non-human hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells toward a specific cell lineage. T lymphocytes, for example, derived from these cultures respond normally to a variety of stimuli and express the diversity of markers expected of mature T cells.

This system provides significant advantages over existing techniques. For example, it can provide for the rapid generation of a large number of differentiated progeny necessary for laboratory analysis and/or therapeutic uses, including for *in vitro* testing of potential gene therapy strategies or for re-infusion into subjects *in vivo.* The matrix itself can be implanted into subjects for *in vivo* studies of hematopoietic cell growth. The system also can reasonably replicate the complex process of hematopoietic cell maintenance, expansion and/or differentiation toward a specific lineage.

Surprisingly, according to the invention, it has been discovered that hematopoietic progenitor cells co-cultured with lymphoreticular stromal cells in a porous solid scaffold, without the addition of exogenous growth agents, generate at a fast rate an unexpectedly high number of functional, differentiated progeny of a lymphoid-specific lineage. The lymphoid tissue from which lymphoreticular stromal cells are derived helps determine the lineage commitment hematopoietic progenitor cells undertake, resulting in the lineage-specificity of the differentiated progeny. Also surprising, according to the invention, is the discovery that lesser amounts of nonlymphoid cells (i.e. myelo-monocytic cells) are generated from the co-culture of hematopoietic progenitor cells and lymphoreticular stromal cells in a porous solid scaffold of the invention when compared to existing methodology. Thus, the present invention permits for the rapid generation of a large number of differentiated, lymphoid-specific cells from a relatively small number of hematopoietic progenitor cells. Such results were never before realized using known art methodologies (e.g., as in U. S. Patent No. 5,677,139 by Johnson et al., which describes the *in vitro* differentiation of CD3+ cells on primate thymic stroma monolayers, or as in U. S. Patent No. 5,541,107 by Naughton et al., which describes a three-dimensional bone marrow cell and tissue culture system).

According to one aspect of the invention, a method for *in vitro* production of lymphoid tissue-specific cells is provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and an amount of lymphoreticular stromal cells into a porous, solid matrix having interconnected pores of a pore size sufficient to permit the hematopoietic progenitor cells and the lymphoreticular stromal cells to grow throughout the matrix. The hematopoietic progenitor cells and the lymphoreticular stromal cells are then co-cultured. The amount of the lymphoreticular stromal cells utilized is sufficient to support the growth and differentiation of the hematopoietic progenitor cells. In one embodiment, co-culturing occurs under conditions sufficient to produce at least a 10-fold increase in the number of lymphoid tissue origin cells. In preferred embodiments, co-culturing occurs under conditions sufficient to produce at least a 20, 50, 100, 200, 300 or 400 -fold increase in the number of lymphoid tissue origin cells. In some embodiments, after the co-culturing, harvesting of the lymphoid tissue origin cells may be performed.

In certain embodiments, the hematopoietic progenitor cells derived from tissue selected from the group consisting of bone marrow, peripheral blood, umbilical cord blood (including mobilized peripheral blood), placental blood, fetal liver, non-human embryonic cells, lymphoid soft tissue and non-human aortal-gonadal-mesonephros derived cells may be pluripotent stem cells, multipotent progenitor cells and/or progenitor cells committed to specific hematopoietic lineages. The progenitor cells committed to specific hematopoietic lineages may be of T cell lineage, B cell lineage, dendritic cell lineage, Langerhans cell lineage and/or lymphoid tissue-specific macrophage cell lineage.

The hematopoietic progenitor cells may be derived from a tissue such as bone marrow, peripheral blood (including mobilized peripheral blood), umbilical cord blood, placental blood, fetal liver, non-human embryonic cells (including non-human embryonic stem cells), non-human aortal-gonadalmesonephros derived cells, and lymphoid soft tissue. Lymphoid soft tissue includes the thymus, spleen, liver, lymph node, skin, tonsil and Peyer's patches. In other embodiments, the lymphoreticular stromal cells may be also derived from at least one of the foregoing lymphoid soft tissues. In preferred embodiments, the lymphoreticular stromal cells are thymic stromal cells and the multipotent progenitor cells and/or committed progenitor cells are committed to a T cell lineage. In other embodiments, the hematopoietic progenitor cells and/or the lymphoreticular stromal cells may be genetically altered.

In one important embodiment of the invention, the hematopoietic progenitor cells are of human origin and the lymphoreticular stromal cells are also of human origin. In another embodiment, the hematopoietic progenitor cells are of human origin and the lymphoreticular stromal cells are of non-human origin. In preferred embodiments, non-human lymphoreticular stromal cells are of murine origin.

In certain embodiments, the lymphoreticular stromal cells are seeded to the matrix at the same time as the hematopoietic progenitor cells. In other embodiments, the lymphoreticular stromal cells are seeded to the matrix prior to inoculating the hematopoietic progenitor cells.

The porous matrix can be one that is an open cell porous matrix having a percent open space of at least 50%, and preferably at least 75%. In one embodiment the porous solid matrix has pores defined by interconnecting ligaments having a diameter at midpoint, on average, of less than 150 µm. Preferably the porous solid matrix is a metal-coated reticulated open cell foam of carbon containing material, the metal coating being selected from the group consisting of tantalum, titanium, platinum (including other metals of the platinum group), niobium, hafnium, tungsten, and combinations thereof. In preferred embodiments, whether the porous solid matrix is metal-coated or not, the matrix is coated with a biological agent selected from the group consisting of collagens, fibronectins, laminins, integrins, angiogenic factors, anti-inflammatory factors, glycosaminoglycans, vitrogen, antibodies and fragments thereof, functional equivalents of these factors (including fragments thereof), and combinations thereof. Most preferably the metal coating is tantalum coated with a biological agent. In certain other embodiments, the porous solid matrix having seeded hematopoietic progenitor cells and their progeny, and lymphoreticular stromal cells, is impregnated with a gelatinous agent that occupies pores of the matrix.

The preferred embodiments of the invention are solid, unitary macrostructures, i.e. not beads or packed beads. They also involve nonbiodegradable materials.

According to any of the foregoing embodiments, the method of the invention can include culturing the cells in an environment that is free of hematopoietic progenitor cell survival and proliferation factors such as interleukins 3, 6 and 11, stem cell ligand and FLT-3 ligand. Still another embodiment of the invention is performing the co-culturing of the hematopoietic progenitor cells and the lymphoreticular stromal cells in an environment that is free altogether of stromal cell conditioned medium and exogenously added hematopoietic growth factors that promote hematopoietic cell maintenance, expansion and/or differentiation, other than serum.

As will be understood, according to the invention, it is possible now to co-culture hematopoietic progenitor cells and lymphoreticular stromal cells in an environment that is free of exogenously added hematopoietic growth factors that promote hematopoietic cell maintenance, expansion and/or differentiation for as little as 7 days and to obtain large numbers of differentiated progeny of a specific lineage.

According to any of the foregoing embodiments, the method of the invention can include co-culturing of the hematopoietic progenitor cells and the lymphoreticular stromal cells with an exogenously added agent selected from the group consisting of stromal cell conditioned medium, and a hematopoietic growth factor that promotes hematopoietic cell maintenance, expansion and/or differentiation, and influences cell localization. In certain embodiments, the hematopoietic growth factor that promotes hematopoietic cell maintenance, expansion and/or differentiation, and influences cell localization, may be an agent that includes interleukin 3, interleukin 6, interleukin 7, interleukin 11, interleukin 12, stem cell factor, FLK-2 ligand, FLT-2 ligand, Epo, Tpo, GMCSF, GCSF, Oncostatin M, and MCSF.

According to another aspect of the invention, a method for *in vivo* maintenance, expansion and/or differentiation of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells is provided. The method involves implanting into a subject a porous, solid matrix having seeded therein hematopoietic progenitor cells (which may include their progeny) and lymphoreticular stromal cells. The porous matrix has interconnected pores of a pore size sufficient to permit the cells to grow throughout the matrix and is an open cell porous matrix having a percent open space of at least 50%, and preferably at least 75%. Various embodiments are provided, wherein the porous solid matrix has one or more of the preferred characteristics as described above.

In certain embodiments, hematopoietic progenitor cells (that may include progeny) and lymphoreticular stromal cells are attached to the matrix by introducing in *vitro* an amount of hematopoietic progenitor cells and an amount of lymphoreticular stromal cells into the porous solid matrix, and co-culturing the hematopoietic progenitor cells in an environment that is free of stromal cell conditioned medium and free of exogenously added hematopoietic growth factors that promote hematopoietic cell maintenance, expansion and/or differentiation, other than serum. Various other embodiments are provided, wherein the co-culturing is performed under conditions as described above. In yet other embodiments, the porous solid matrix having seeded hematopoietic progenitor cells (that may include progeny) and lymphoreticular stromal cells is impregnated with a gelatinous agent that occupies pores of the matrix.

According to one aspect of the invention, a method for inducing T cell anergy *in vitro* is provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, an amount of antigen presenting cells, and an amount of lymphoreticular stromal cells into a porous, solid matrix having interconnected pores of a pore size sufficient to permit the hematopoietic progenitor cells and the lymphoreticular stromal cells to grow throughout the matrix, and co-culturing the hematopoietic progenitor cells, the antigen presenting cells and the lymphoreticular stromal cells in the presence of at least one antigen under conditions sufficient to induce the formation of T cells and/or T cell progenitors and to inhibit activation of the formed T cells and/or T cell progenitors.

In certain embodiments, the hematopoietic progenitor cells may be pluripotent stem cells, multipotent progenitor cells and/or progenitor cells committed to specific hematopoietic lineages. The hematopoietic progenitor cells may be derived from a tissue such as bone marrow, peripheral blood (including mobilized peripheral blood), umbilical cord blood, placental blood, fetal liver, non-human embryonic cells (including non-human embryonic stem cells), non-human aortal-gonadal-mesonephros derived cells, and lymphoid soft tissue. Lymphoid soft tissue includes the thymus, spleen, liver, lymph node, skin, tonsil and/or Peyer's patches. In other embodiments, the lymphoreticular stromal cells may be also derived from at least one of the foregoing lymphoid soft tissues. In preferred embodiments, the lymphoreticular stromal cells are thymic stromal cells and the multipotent progenitor cells and/or committed progenitor cells are committed to a T cell lineage. In other embodiments, the hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells and/or the lymphoreticular stromal cells may be genetically altered. In certain embodiments, the antigen presenting cells include cells such as dendritic cells, monocytes/macrophages, Langerhans cells, Kupfer cells, microglia, alveolar macrophages and B cells. In other embodiments, the antigen presenting cells are derived from hematopoietic progenitor cells *in vitro,* provided said hematopoietic proganitor cells are not derived from human embryos. Various embodiments are provided, wherein the porous solid matrix has one or more of the preferred characteristics as described above.

According to another aspect of the invention, a method for inducing T cell reactivity *in vitro* is provided. The method involves introducing an amount of hematopoietic progenitor cells derived from tissue selected from the group consisting of bone marrow, peripheral blood (including mobilized nerinheral blood), umbilical cord blood, placental blood, fetal liver, non-human embryonic cells, lymphoid soft tissue and non-human aortal-gonadal-mesonephros derived cells, an amount of antigen presenting cells, and an amount of lymphoreticular stromal cells into a porous, solid matrix having interconnected pores of a pore size sufficient to permit the hematopoietic progenitor cells and the lymphoreticular stromal cells to grow throughout the matrix, and co-culturing the hematopoietic progenitor cells, the antigen presenting cells and the lymphoreticular stromal cells in the presence of at least one antigen under conditions sufficient to induce the formation of T cells and/or T cell progenitors from the hematopoietic progenitor cells having specificity for the at least one antigen. Various embodiments are provided, wherein the hematopoietic progenitor cells, the lymphoreticular stromal cells, and the porous solid matrix have one or more of the preferred characteristics as described above, and the cells are cultured as described above. In certain embodiments, the antigen presenting cells include cells such as dendritic cells, monocytes/macrophages, Langerhans cells, Kupfer cells, microglia, alveolar macrophages and B cells. In other embodiments, the antigen presenting cells are derived from hematopoietic progenitor cells in *vitro*, provided said hematopoietic cells are not derived from human embryos. In further embodiments, the method further comprises administering a co-stimulatory agent to the co-culture. Preferred co-stimulatory agents include lymphocyte function associated antigen 3 (LFA-3), CD2, CD40, CD80/B7-1, CD86/B7-2, OX-2, CD70, and CD82.

In yet another aspect of the invention, a solid porous matrix is provided wherein hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, with or without their progeny, and lymphoreticular stromal cells are attached to the solid porous matrix. The lymphoreticular stromal cells are present in an amount sufficient to support the growth and differentiation of hematopoietic progenitor cells. In certain embodiments, the hematopoietic progenitor cells are attached to the lymphoreticular stromal cells. The porous matrix can be one that is an open cell porous matrix having a percent open space of at least 50%, and preferably at least 75%. In one embodiment the porous solid matrix has pores defined by interconnecting ligaments having a diameter at midpoint, on average, of less than 150 µm. Preferably the porous solid matrix is a metal-coated reticulated open cell foam of carbon containing material, the metal coating being selected from the group consisting of tantalum, titanium, platinum (including other metals of the platinum group), niobium, hafnium, tungsten, and combinations thereof. In preferred embodiments, whether the porous solid matrix is metal-coated or not, the matrix is coated with a biological agent selected from the group consisting of collagens, fibronectins, laminins, integrins, angiogenic factors, anti-inflammatory factors, glycosaminoglycans, vitrogen, antibodies and fragments thereof, functional equivalents of these factors, and combinations thereof. Most preferably the metal coating is tantalum coated with a biological agent. In certain other embodiments the porous solid matrix having seeded hematopoietic progenitor cells and lymphoreticular stromal cells, is impregnated with a gelatinous agent that occupies pores of the matrix.

In a further aspect of the invention, a method for identifying an agent suspected of affecting hematopoietic cell development is provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and an amount of lymphoreticular stromal cells into a porous, solid matrix having interconnected pores of a pore size sufficient to permit the hematopoietic progenitor cells and the lymphoreticular stromal cells to grow throughout the matrix, co-culturing the hematopoietic progenitor cells and the lymphoreticular stromal cells in the presence of at least one candidate agent suspected of affecting hematopoietic cell development (in a test co-culture), and determining whether the at least one candidate agent affects hematopoietic cell development in the test co-culture by comparing the test co-culture hematopoietic cell development to a control co-culture, whereby hematopoietic progenitor cells and lymphoreticular stromal cells are co-cultured in the absence of the at least one candidate agent. Various embodiments are provided, wherein the hematopoietic progenitor cells, the lymphoreticular stromal cells, and the porous solid matrix have one or more of the preferred characteristics as described above, and the cells are cultured as described above. In certain embodiments, hematopoietic progenitor cell development includes hematopoietic progenitor cell maintenance, expansion, differentiation toward a specific cell lineage, and/or cell-death (including apoptosis). In preferred embodiments the lymphoreticular stromal cells are thymic stromal cells.

In another aspect of the invention, a method for isolating from a cell culture an agent suspected of affecting hematopoietic cell development is provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells and an amount of lymphoreticular stromal cells into a porous, solid matrix having interconnected pores of a pore size sufficient to permit the hematopoietic progenitor cells and the lymphoreticular stromal cells to grow throughout the matrix, co-culturing the hematopoietic progenitor cells and the lymphoreticular stromal cells, obtaining a test-supernatant from the co-culture, comparing the test-supernatant to a control-supernatant, and obtaining a subfraction of the test-supernatant that contains an agent suspected of affecting hematopoietic cell development that is absent from the control-supernatant. In certain embodiments the agent suspected of affecting hematopoietic cell development may be present in the control-supernatant and absent from the test-supernatant. In other embodiments, the agent suspected of affecting hematopoietic cell development in one supernatant may be different to an agent suspected of affecting hematopoietic cell development in the other supernatant (e.g., in size, via a post-translational modification, in an alternatively spliced variant form, etc.). Various embodiments are provided, wherein the hematopoietic progenitor cells, the lymphoreticular stromal cells, and the porous solid matrix have one or more of the preferred characteristics as described above, and the cells are cultured as described above. In certain embodiments, hematopoietic progenitor cell development includes hematopoietic progenitor cell maintenance, expansion, differentiation toward a specific cell lineage, and/or cell-death (including apoptosis). In preferred embodiments, the lymphoreticular stromal cells are thymic stromal cells. In certain other embodiments, the control culture system of the prior art (where the control-supernatant can be obtained from) is the one described in U.S. Patent No. 5,677,139 by Johnson et al*.*

These and other aspects of the invention, as well as various advantages and utilities, will be more apparent with reference to the detailed description of the preferred embodiments.

### Brief Description of the Drawings

Figure 1 shows the differentiation of human CD34⁺ progenitor cells into T cells, in co-culture with murine thymic stroma cells on a three-dimensional matrix; the data in Fig. 1(a) shows the acquisition of CD2 and the down-regulation of the hematopoietic progenitor cell marker CD34; the data in Fig. 1(b) shows the discrete populations of SP CD4⁺ and SP CD8⁺ cells, including their DP CD4⁺CD8⁺ precursors; the data in Figs. 1(c and d) shows that all CD4⁺(c) and CD8⁺(d) cells co-expressed CD3.
Figure 2 shows the intrasample variability in numbers of T cells generated in a co-culture system of the invention.
Figure 3 shows the intersample variability in numbers of T cells generated in a co-culture system of the invention.

### Detailed Description of the Invention

The invention involves the unexpected discovery that hematopoietic progenitor cells co-cultured with lymphoreticular stromal cells in a porous solid scaffold, without the addition of exogenous growth agents, generate at a fast rate an unexpectedly high number of functional, differentiated progeny of a lymphoid tissue-specific lineage. Also surprising, according to the invention, has been the discovery that lesser amounts of nonlymphoid cells (i.e. myelo-monocytic cells) are generated from the co-culture of hematopoietic progenitor cells and lymphoreticular stromal cells in a porous solid scaffold of the invention when compared to existing technology. Thus, the present invention, and in contrast to what has been previously achieved in the art, permits for the rapid generation of a large number of differentiated, lymphoid-specific cells from a relatively small number of hematopoietic progenitor cells.

Methods of the invention are therefore useful *inter alia* for establishing immunocompetence in patients suffering from an immunodeficiency, e.g., a T cell or B cell deficiency, e.g., a thymic based immunodeficiency, e.g., a congenital immunodeficiency due to thymic aplasia or dysfunction, an acquired immune disorder, e.g., AIDS, immunoincompetence resulting form a neoplastic disease, or immunoincompetence resulting from a medical procedure, e.g., chemotherapy, immunocompetence in response to an antigen, etc.

The invention in one aspect involves culturing hematopoietic cells in a porous solid matrix, in the absence of exogenous growth agents, to produce lymphoid tissue origin (lymphoid tissue-specific) cells.

A porous, solid matrix, is defined as a three-dimensional structure with "sponge-like" continuous pores forming an interconnecting network. The matrix can be rigid or elastic, and it provides a scaffold upon which cells can grow throughout. Its pores are interconnected and provide the continuous network of channels extending through the matrix and also permit the flow of nutrients throughout. A preferred matrix is an open cell foam matrix having a percent open space of at least 50% and preferably 75%. Thus, it is preferred that the open space comprise the majority of the matrix. This is believed to maximize cell migration, cell-cell contact, space for cell growth and accessibility to nutrients. It is preferred that the porous matrix be formed of a reticulated matrix of ligaments which at their center point are less than 150µm in diameter, preferably 60µm, whereby a cell can reside on or interact with a portion of the ligament. Preferably, the average pore diameter is on the order of 300µm, which resembles cancellous bone. Suitable matrices can be obtained using a number of different methods. Examples of such methods include solvent casting or extraction of polymers, track etching of a variety of materials, foaming of a polymer, the replamineform process for hydroxyapatite, and other methodologies well known to those of ordinary skill in the art. The materials employed can be natural or synthetic, including biological materials such as proteins, hyaluronic acids, synthetic polymers such as polyvinyl pyrolidones, polymethylmethacrylate, methyl cellulose, polystyrene, polypropylene, polyurethane, ceramics such as tricalcium phosphate, calcium aluminate, calcium hydroxyapatite and ceramic-reinforced or coated polymers. If the starting material for the scaffold is not metal, a metal coating can be applied to the three-dimensional matrix. Metal coatings provide further structural support and/or cell growth and adhesive properties to the matrix. Preferred metals used as coatings comprise tantalum, titanium, platinum and metals in the same element group as platinum, niobium, hafnium, tungsten, and combinations of alloys thereof. Coating methods for metals include a process such as CVD (Chemical Vapor Deposition).

The preferred matrix, refered to herein throughout as Cellfoam (Cytomatrix, Woburn, MA), is described in detail in U.S. Patent No. 5,282,861, and is incorporated herein by reference. More specifically, the preferred matrix is a reticulated open cell substrate formed by a lightweight, substantially rigid foam of carbon-containing material having open spaces defined by an interconnecting network, wherein said foam material has interconnected continuous channels, and a thin film of metallic material deposited onto the reticulated open cell substrate and covering substantially all of the interconnecting network to form a composite porous biocompatible material creating a porous microstructure similar to that of natural cancellous bone.

Additionally, such matrices can be coated with biological agents which can promote cell adhesion for the cultured hematopoietic progenitor cells, allowing for improved migration, growth and proliferation. Moreover, when these matrices are used for the *in vivo* maintenance, expansion and/or differentiation of hematopoietic progenitor cells (i.e., when the matrices with the cells are implanted into a subject, -see also discussion below), biological agents that promote angiogenesis (vascularization) and biological agents that prevent/reduce inflammation may also be used for coating of the matrices. Preferred biological agents comprise collagens, fibronectins, laminins, integrins, angiogenic factors, anti-inflammatory factors, glycosaminoglycans, vitrogen, antibodies and fragments thereof, functional equivalents of these agents, and combinations thereof.

Angiogenic factors include platelet derived growth factor (PDGF), vascular endothelial growth factor (VEGF), basic fibroblast growth factor (bFGF), bFGF-2, leptins, plasminogen activators (tPA, uPA), angiopoietins, lipoprotein A, transforming growth factor-β, bradykinin, angiogenic oligosaccharides (e.g., hyaluronan, heparan sulphate), thrombospondin, hepatocyte growth factor (also known as scatter factor) and members of the CXC chemokine receptor family. Anti-inflammatory factors comprise steroidal and non-steroidal compounds and examples include: Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate ; Rimexolone; Romazarit ; Salcolex ; Salnacedin; Salsalate ; Sanguinarium Chloride ; Seclazone ; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine ; Tiopinac ; Tixocortol Pivalate; Tolmetin; Tolmetin Sodium; Triclonide; Triflumidate; Zidometacin; Zomepirac Sodium.

In certain embodiments of the invention the porous solid matrix having seeded hematopoietic progenitor cells, with or without their progeny, and lymphoreticular stromal cells is impregnated with a gelatinous agent that occupies pores of the matrix. The hematopoietic progenitor cells, with or without their progeny, and/or the lymphoreticular stromal cells can be seeded prior to, substantially at the same time as, or following impregnation (or infiltration) with a gelatinous agent. For example, the cells may be mixed with the agent and seeded at the same time as the the impregnation of the matrix with the agent. In some embodiments, the cells are seeded onto the porous solid matrix prior to application of the agent. In certain embodiments the lymphoreticular stromal cells are seeded in a similar manner. A person of ordinary skill in the art can easily determine seeding conditions. Preferably the lymphoreticular stromal cells are seeded prior to the hematopoietic progenitor cells and prior to impregnation with the agent.

"Impregnation" with a gelatinous agent can serve, *inter alia,* to contain the cells within the matrix, or to help maintain and/or enhance cell attachment onto the matrix. The "gelatinous" agent may be one that can be maintained in a fluid state initially (i.e. gelable), and after its application into the matrix, be gelatinized *in situ* in the matrix. Such gelatinization may occur in a number of different ways, including altering the agent's temperature, irradiating the agent with an energy source (e.g., light), etc. The "gelatinous" agent also is characterized by its ability to allow the nutrients of the growth media to reach the cells throughout the matrix. Exemplary "gelatinous" agents include cellulosic polysaccharides (such as cellulose, hemicellulose, methylcellulose, and the like), agar, agarose, albumin, algal mucin, mucin, mucilage, collagens, glycosaminoglycans, and proteoglycans (including their sulphated forms). In certain embodiments, the gelatinous agent may impregnate the matrix completely, in some embodiments partially, and in other embodiments minimally, serving only as a coating of all or some of the outer surfaces of the matrix. In important embodiments where gelatinous agents are employed, the "gelatinous" agent is methylcellulose and the impregnation is complete.

According to the invention, hematopoietic progenitor cells and lymphoreticular stromal cells are co-cultured in one of the foregoing porous solid matrices, in the absence of exogenous growth agents, to produce lymphoid tissue origin (lymphoid tissue-specific) cells. "Lymphoid tissue origin" (lymphoid tissue-specific) cells, as used herein, refer to cells that may be produced *in vitro* or *in vivo* according to the invention, and are substantially similar (e.g., in properties and function) to the cells produced naturally *in vivo* from organs and tissues that include the bone marrow, thymus, lymph nodes, spleen and mucosal associated lymphoid tissue (unencapsulated tissue lining the respiratory, alimentary and genito-urinary tracts).

"Hematopoietic progenitor cells" as used herein refers to immature blood cells having the capacity to self-renew and to differentiate into the more mature blood cells (also described herein as "progeny") comprising granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), and monocytes (e.g., monocytes, macrophages). It is known in the art that such cells may or may not include CD34⁺ cells. CD34⁺ cells are immature cells present in the "blood products" described below, express the CD34 cell surface marker, and are believed to include a subpopulation of cells with the "progenitor cell" properties defined above. It is well known in the art that hematopoietic progenitor cells include pluripotent stem cells, multipotent progenitor cells (e.g., a lymphoid stem cell), and/or progenitor cells committed to specific hematopoietic lineages. The progenitor cells committed to specific hematopoietic lineages may be of T cell lineage, B cell lineage, dendritic cell lineage, Langerhans cell lineage and/or lymphoid tissuespecific macrophage cell lineage.

The hematopoietic progenitor cells can be obtained from blood products. A "blood product" as used in the present invention defines a product obtained from the body or an organ of the body containing cells of hematopoietic origin. Such sources include unfractionated bone marrow, umbilical cord, peripheral blood, liver, thymus, lymph and spleen. It will be apparent to those of ordinary skill in the art that all of the aforementioned crude or unfractionated blood products can be enriched for cells having "hematopoietic progenitor cell" characteristics in a number of ways. For example, the blood product can be depleted from the more differentiated progeny. The more mature, differentiated cells can be selected against, via cell surface molecules they express. Additionally, the blood product can be fractionated selecting for CD34⁺ cells. As mentioned earlier, CD34⁺ cells are thought in the art to include a subpopulation of cells capable of self-renewal and pluripotentiality. Such selection can be accomplished using, for example, commercially available magnetic anti-CD34 beads (Dynal, Lake Success, NY). Unfractionated blood products can be obtained directly from a donor or retrieved from cryopreservative storage.

The cells co-cultured with the hematopoietic progenitor cells according to the methods of the invention are lymphoreticular stromal cells. "Lymphoreticular stromal cells" as used herein may include, but are not limited to, all cell types present in a lymphoid tissue which are not lymphocytes or lymphocyte precursors or progenitors, e.g., epithelial cells, endothelial cells, mesothelial cells, dendritic cells, splenocytes and macrophages. Lymphoreticular stromal cells also include cells that would not ordinarily function as lymphoreticular stromal cells, such as fibroblasts, which have been genetically altered to secrete or express on their cell surface the factors necessary for the maintenance, growth and/or differentiation of hematopoietic progenitor cells, including their progeny. Lymphoreticular stromal cells are derived from the disaggregation of a piece of lymphoid tissue (see discussion below and the Examples). Such cells according to the invention are capable of supporting *in vitro* the maintenance, growth and/or differentiation of hematopoietic progenitor cells, including their progeny. By "lymphoid tissue" it is meant to include bone marrow, peripheral blood (including mobilized peripheral blood), umbilical cord blood, placental blood, fetal liver, non-human embryonic cells (including non-human embryonic stem cells), non-human aortal-gonadal-mesonephros derived cells, and lymphoid soft tissue. "Lymphoid soft tissue" as used herein includes, but is not limited to, tissues such as thymus, spleen, liver, lymph node, skin, tonsil, adenoids and Peyer's patch, and combinations thereof.

Lymphoreticular stromal cells provide the supporting microenvironment in the intact lymphoid tissue for the maintenance, growth and/or differentiation of hematopoietic progenitor cells, including their progeny. The microenvironment includes soluble and cell surface factors expressed by the various cell types which comprise the lymphoreticular stroma. Generally, the support which the lymphoreticular stromal cells provide may be characterized as both contact-dependent and non-contact-dependent.

Lymphoreticular stromal cells may be allogeneic, syngeneic or xenogeneic with respect to the hematopoietic progenitor cells. Lymphoreticular stroma cells may be obtained from the lymphoid tissue of a human or a non-human subject at any time after the organ/tissue has developed to a stage (i.e., the maturation stage) at which it can support the maintenance growth and/or differentiation of hematopoietic progenitor cells. The stage will vary between organs/tissues and between subjects. In primates, for example, the maturation stage of thymic development is achieved during the second trimester. At this stage of development the thymus can produce peptide hormones such as thymulin, α₁ and β₄ -thymosin, and thymopoietin, as well as other factors required to provide the proper microenvironment for T cell differentiation. The different maturation stages for the different organs/tissues and between different subjects are well known in the art.

The lymphoid tissue from which lymphoreticular stromal cells are derived usually determines the lineage-commitment hematopoietic progenitor cells undertake, resulting in the lineage-specificity of the differentiated progeny. In certain embodiments, the lymphoreticular stromal cells are thymic stromal cells and the multipotent progenitor cells and/or committed progenitor cells are committed to a T cell lineage. In other embodiments, the lymphoreticular stromal cells may be splenic stromal cells and the multipotent progenitor cells and/or committed progenitor cells are committed to a B cell lineage. Also surprising, according to the invention, has been the discovery that the highest yield of differentiated progeny occurs when human hematopoietic progenitor cells are cultured in the presence of xenogeneic (non-human) lymphoreticular stromal cells. Preferably the xenogeneic lymphoreticular stromal cells are of murine origin.

Unexpectedly, it has also been discovered that lesser amounts of nonlymphoid-specific cells (i.e. myelo-monocytic cells) are generated from the foregoing co-cultures when compared to existing methodology. In other words, more homogeneous differentiation of cells with fewer contaminant cell types (nonlymphoid) is observed form cultures of the present invention on Cellfoam, enabling the preservation of immature progenitors (CD34⁺ cells) while promoting the differentiation of more mature T progeny.

Various other embodiments are provided, wherein the lymphoreticular stromal cells may be genetically altered. The lymphoreticular stromal cells may be transfected with exogenous DNA that encodes, for example, one of the hematopoietic growth factors described above (see fibroblast discussion above).

As mentioned earlier, lymphoreticular stromal cells are derived from the disaggregation of a piece of lymphoid tissue, forming cell suspensions. Preferably, single cell suspensions are generated. These lymphoreticular stromal cell suspensions may be used directly, or made non-mitotic by procedures standard in the tissue culture art. Examples of such methods are irradiation of lymphoreticular stromal cells with a gamma-ray source or incubation of the cells with mitomycin C for a sufficient amount of time to render the cells mitotically inactive. Mitotic inactivation is preferred when the lymphoreticular stromal cells are of human origin (to eliminate progenitor cells that may be present in the suspension). The lymphoreticular stromal cells may then be seeded into a three-dimensional matrix of the invention and permitted to attach to a surface of the porous, solid matrix. It should be noted that the lymphoreticular stromal cells may alternatively be cryopreserved for later use or for storage and shipment to remote locations, such as for use in connection with the sale of kits. Cryopreservation of cells cultured *in vitro* is well established in the art. Subsequent to isolation (and/or mitotic inactivation) of a cell sample, cells may be cryopreserved by first suspending the cells in a cryopreservation medium and then gradually freezing the cell suspension. Frozen cells are typically stored in liquid nitrogen or at an equivalent temperature in a medium containing serum and a cryopreservative such as dimethyl sulfoxide.

The co-culture of the hematopoietic progenitor cells (and progeny thereof) with lymphoreticular stromal cells, preferably occurs under conditions sufficient to produce a percent increase in the number of lymphoid tissue origin cells deriving from the hematopoietic progenitor cells. The conditions used refer to a combination of conditions known in the art (e.g., temperature, CO₂ and O₂ content, nutritive media, time-length, etc.). The time sufficient to increase the number of cells is a time that can be easily determined by a person skilled in the art, and can vary depending upon the original number of cells seeded. The amounts of hematopoietic progenitor cells and lymphoreticular stromal cells initially introduced (and subsequently seeded) into the porous solid matrix may vary according to the needs of the experiment. The ideal amounts can be easily determined by a person skilled in the art in accordance with needs. Preferably, the lymphoreticular stromal cells would form a confluent layer onto the matrix. Hematopoietic progenitor cells may be added at different numbers. As an example, discoloration of the media over a certain period of time can be used as an indicator of confluency. Additionally, and more precisely, different numbers of hematopoietic progenitor cells or volumes of the blood product can be cultured under identical conditions, and cells can be harvested and counted over regular time intervals, thus generating the "control curves". These "control curves" can be used to estimate cell numbers in subsequent occasions (see the Examples section).

The conditions for determining colony forming potential are similarly determined. Colony forming potential is the ability of a cell to form progeny. Assays for this are well known to those of ordinary skill in the art and include seeding cells into a semi-solid matrix, treating them with growth factors, and counting the number of colonies.

In preferred embodiments of the invention, the hematopoietic progenitor cells may be harvested. "Harvesting" hematopoietic progenitor cells is defined as the dislodging or separation of cells from the matrix. This can be accomplished using a number of methods, such as enzymatic and non-enzymatic, centrifugal, electrical or by size, or the one preferred in the present invention, by flushing of the cells using the media in which the cells are incubated. The cells can be further collected, separated, and further expanded generating even larger populations of differentiated progeny.

As mentioned above, the hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and progeny thereof, can be genetically altered. Genetic alteration of a hematopoietic progenitor cell includes all transient and stable changes of the cellular genetic material which are created by the addition of exogenous genetic material. Examples of genetic alterations include any gene therapy procedure, such as introduction of a functional gene to replace a mutated or nonexpressed gene, introduction of a vector that encodes a dominant negative gene product, introduction of a vector engineered to express a ribozyme and introduction of a gene that encodes a therapeutic gene product. Natural genetic changes such as the spontaneous rearrangement of a T cell receptor gene without the introduction of any agents are not included in this concept. Exogenous genetic material includes nucleic acids or oligonucleotides, either natural or synthetic, that are introduced into the hematopoietic progenitor cells. The exogenous genetic material may be a copy of that which is naturally present in the cells, or it may not be naturally found in the cells. It typically is at least a portion of a naturally occurring gene which has been placed under operable control of a promoter in a vector construct.

The invention involves the unexpected discovery that hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells can be more efficiently genetically altered if the genetic alteration occurs while the hematopoietic progenitor cells are on and within a solid porous matrix as described above.

Various techniques may be employed for introducing nucleic acids into cells. Such techniques include transfection of nucleic acid-CaPO₄ precipitates, transfection of nucleic acids associated with DEAE, transfection with a retrovirus including the nucleic acid of interest, liposome mediated transfection, and the like. For certain uses, it is preferred to target the nucleic acid to particular cells. In such instances, a vehicle used for delivering a nucleic acid according to the invention into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. For example, where liposomes are employed to deliver the nucleic acids of the invention, proteins which bind to a surface membrane protein associated with endocytosis may be incorporated into the liposome formulation for targeting and/or to facilitate uptake. Such proteins include proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids.

In the present invention, the preferred method of introducing exogenous genetic material into hematopoietic cells is by transducing the cells *in situ* on the matrix using replication-deficient retroviruses. Replication-deficient retroviruses are capable of directing synthesis of all virion proteins, but are incapable of making infectious particles. Accordingly, these genetically altered retroviral vectors have general utility for high-efficiency transduction of genes in cultured cells, and specific utility for use in the method of the present invention. Retroviruses have been used extensively for transferring genetic material into cells. Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with the viral particles) are provided in the art.

The major advantage of using retroviruses is that the viruses insert efficiently a single copy of the gene encoding the therapeutic agent into the host cell genome, thereby permitting the exogenous genetic material to be passed on to the progeny of the cell when it divides. In addition, gene promoter sequences in the LTR region have been reported to enhance expression of an inserted coding sequence in a variety of cell types. The major disadvantages of using a retrovirus expression vector are (1) insertional mutagenesis, i.e., the insertion of the therapeutic gene into an undesirable position in the target cell genome which, for example, leads to unregulated cell growth and (2) the need for target cell proliferation in order for the therapeutic gene carried by the vector to be integrated into the target genome. Despite these apparent limitations, delivery of a therapeutically effective amount of a therapeutic agent via a retrovirus can be efficacious if the efficiency of transduction is high and/or the number of target cells available for transduction is high.

Yet another viral candidate useful as an expression vector for transformation of hematopoietic cells is the adenovirus, a double-stranded DNA virus. Like the retrovirus, the adenovirus genome is adaptable for use as an expression vector for gene transduction, i.e., by removing the genetic information that controls production of the virus itself. Because the adenovirus functions usually in an extrachromosomal fashion, the recombinant adenovirus does not have the theoretical problem of insertional mutagenesis. On the other hand, adenoviral transformation of a target hematopoietic cell may not result in stable transduction. However, more recently it has been reported that certain adenoviral sequences confer intrachromosomal integration specificity to carrier sequences, and thus result in a stable transduction of the exogenous genetic material.

Thus, as will be apparent to one of ordinary skill in the art, a variety of suitable vectors are available for transferring exogenous genetic material into hematopoietic cells. The selection of an appropriate vector to deliver a therapeutic agent for a particular condition amenable to gene replacement therapy and the optimization of the conditions for insertion of the selected expression vector into the cell, are within the scope of one of ordinary skill in the art without the need for undue experimentation. The promoter characteristically has a specific nucleotide sequence necessary to initiate transcription. Optionally, the exogenous genetic material further includes additional sequences (i.e., enhancers) required to obtain the desired gene transcription activity. For the purpose of this discussion an "enhancer" is simply any nontranslated DNA sequence which works contiguous with the coding sequence (in cis) to change the basal transcription level dictated by the promoter. Preferably, the exogenous genetic material is introduced into the hematopoietic cell genome immediately downstream from the promoter so that the promoter and coding sequence are operatively linked so as to permit transcription of the coding sequence. A preferred retroviral expression vector includes an exogenous promoter element to control transcription of the inserted exogenous gene. Such exogenous promoters include both constitutive and inducible promoters.

Naturally-occurring constitutive promoters control the expression of essential cell functions. As a result, a gene under the control of a constitutive promoter is expressed under all conditions of cell growth. Exemplary constitutive promoters include the promoters for the following genes which encode certain constitutive or "housekeeping" functions: hypoxanthine phosphoribosyl transferase (HPRT), dihydrofolate reductase (DHFR) (Scharfmann et al., Proc. Natl. Acad. Sci. USA 88:4626-4630 (1991)), adenosine deaminase, phosphoglycerol kinase (PGK), pyruvate kinase, phosphoglycerol mutase, the actin promoter (Lai et al., Proc. Natl. Acad. Sci. USA 86: 10006-10010 (1989)), and other constitutive promoters known to those of skill in the art. In addition; many viral promoters function constitutively in eukaryotic cells. These include: the early and late promoters of SV40; the long terminal repeats (LTRS) of Moloney Leukemia Virus and other retroviruses; and the thymidine kinase promoter of Herpes Simplex Virus, among many others. Accordingly, any of the above-referenced constitutive promoters can be used to control transcription of a heterologous gene insert.

Genes that are under the control of inducible promoters are expressed only or to a greater degree, in the presence of an inducing agent, (e.g., transcription under control of the metallothionein promoter is greatly increased in presence of certain metal ions). Inducible promoters include responsive elements (REs) which stimulate transcription when their inducing factors are bound. For example, there are REs for serum factors, steroid hormones, retinoic acid and cyclic AMP. Promoters containing a particular RE can be chosen in order to obtain an inducible response and in some cases, the RE itself may be attached to a different promoter, thereby conferring inducibility to the recombinant gene. Thus, by selecting the appropriate promoter (constitutive versus inducible; strong versus weak), it is possible to control both the existence and level of expression of a therapeutic agent in the genetically modified hematopoietic cell. Selection and optimization of these factors for delivery of a therapeutically effective dose of a particular therapeutic agent is deemed to be within the scope of one of ordinary skill in the art without undue experimentation, taking into account the above-disclosed factors and the clinical profile of the patient.

In addition to at least one promoter and at least one heterologous nucleic acid encoding the therapeutic agent, the expression vector preferably includes a selection gene, for example, a neomycin resistance gene, for facilitating selection of hematopoietic cells that have been transfected or transduced with the expression vector. Alternatively, the hematopoietic cells are transfected with two or more expression vectors, at least one vector containing the gene(s) encoding the therapeutic agent(s), the other vector containing a selection gene. The selection of a suitable promoter, enhancer, selection gene and/or signal sequence (described below) is deemed to be within the scope of one of ordinary skill in the art without undue experimentation.

The selection and optimization of a particular expression vector for expressing a specific gene product in an isolated hematopoietic cell is accomplished by obtaining the gene, preferably with one or more appropriate control regions (e.g., promoter, insertion sequence); preparing a vector construct comprising the vector into which is inserted the gene; transfecting or transducing cultured hematopoietic cells *in vitro* with the vector construct; and determining whether the gene product is present in the cultured cells.

**Table 1. Human Gene Therapy Protocols Approved by RAC: 1990-1994**

| Severe combined immune deficiency (SCID) due to ADA deficiency | Autologous lymphocytes transduced with human ADA gene | 7/31/90 |
|---|---|---|
| Advanced cancer | Tumor-infiltrating lymphocytes transduced with tumor necrosis factor gene | 7/31/90 |
| Advanced cancer | Immunization with autologous cancer cells transduced with tumor necrosis factor gene | 10/07/91 |
| Advanced cancer | Immunization with autologous cancer cells transduced with interleukin-2 gene | 10/07/91 |
| Asymptomatic patients infected with HIV-1 | Murine Retro viral vector encoding HIV-I genes [HIV-IT(V)] | 6/07/93 |
| AIDS | Effects of a transdominant form of *rev* gene on AIDS intervention | 6/07/93 |
| Advanced cancer | Human multiple-drug resistance (MDR) gene transfer | 6/08/93 |
| HIV infection | Autologous lymphocytes transduced with catalytic ribozyme that cleaves HIV-1 RNA (Phase I study) | 9/10/93 |
| Metastatic melanoma | Genetically engineered autologous tumor vaccines producing interleukin-2 | 9/10/93 |
| HIV infection | Murine Retro viral vector encoding HIV-IT(V) genes (open label Phase I/II trial) | 12/03/93 |
| HIV infection (identical twins) | Adoptive transfer of syngeneic cytotoxic T lymphocytes (Phase I/II pilot study) | 3/03/94 |
| Breast cancer (chemo-protection during therapy) | Use of modified Retro virus to introduce chemotherapy resistance sequences into normal hematopoietic cells (pilot study) | 6/09/94 |
| Fanconi's anemia | Retro viral mediated gene transfer of the Fanconi anemia complementation group C gene to hematopoietic progenitors | 6/09/94 |
| Metastatic prostate | Autologous human granulocyte macrophage-colony | ORDA/NIH |
| carcinoma | stimulating factor gene transduced prostate cancer vaccine *(first protocol to be approved under the accelerated review process; ORDA=Office of Recombinate DNA Activities) | 8/03/94* |
| Metastatic breast cancer | *In vivo* infection with breast-targeted Retro viral vector expressing antisense *c-fox* or antisense *c-myc* RNA | 9/12/94 |
| Metastatic breast cancer (refractory or recurrent) | Non-viral system (liposome-based) for delivering human interleukin-2 gene into autologous tumor cells (pilot study) | 9/12/94 |
| Mild Hunter syndrome | Retro viral-mediated transfer of the iduronate-2-sulfatase gene into lymphocytes | 9/13/94 |
| Advanced mesothelioma | Use of recombinant adenovirus (Phase I study) | 9/13/94 |

The foregoing (Table 1), represent only examples of genes that can be delivered according to the methods of the invention. Suitable promoters, enhancers, vectors, etc., for such genes are published in the literature associated with the foregoing trials. In general, useful genes replace or supplement function, including genes encoding missing enzymes such as adenosine deaminase (ADA) which has been used in clinical trials to treat ADA deficiency and cofactors such as insulin and coagulation factor VIII. Genes which affect regulation can also be administered, alone or in combination with a gene supplementing or replacing a specific function. For example, a gene encoding a protein which suppresses expression of a particular protein-encoding gene can be administered. The invention is particularly useful in delivering genes which stimulate the immune response, including genes encoding viral antigens, tumor antigens, cytokines (e.g. tumor necrosis factor) and inducers of cytokines (e.g. endotoxin).

Employing the culture conditions described in greater detail below, it is possible according to the invention to preserve hematopoietic progenitor cells and to stimulate the expansion of hematopoietic progenitor cell number and/or colony forming unit potential. Once expanded, the cells, for example, can be returned to the body to supplement, replenish, etc. a patient's hematopoietic progenitor cell population. This might be appropriate, for example, after an individual has undergone chemotherapy. There are certain genetic conditions wherein hematopoietic progenitor cell numbers are decreased, and the methods of the invention may be used in these situations as well.

It also is possible to take the increased numbers of hematopoietic progenitor cells produced according to the invention and stimulate them with hematopoietic growth agents that promote hematopoietic cell maintenance, expansion and/or differentiation, and also influence cell localization, to yield the more mature blood cells, *in vitro.* Such expanded populations of blood cells may be applied *in vivo* as described above, or may be used experimentally as will be recognized by those of ordinary skill in the art. Such differentiated cells include those described above, as well as T cells, plasma cells, erythrocytes, megakaryocytes, basophils, polymorphonuclear leukocytes, monocytes, macrophages, eosinohils and platelets.

In all of the culturing methods according to the invention, except as otherwise provided, the media used is that which is conventional for culturing cells. Examples include RPMI, DMEM, Iscove's, etc. Typically these media are supplemented with human or animal plasma or serum. Such plasma or serum can contain small amounts of hematopoietic growth factors. The media used according to the present invention, however, can depart from that used conventionally in the prior art. In particular, it has been discovered, surprisingly, that hematopoietic progenitor cells can be cultured on the matrices described above for extended periods of time without the need for adding any exogenous growth agents (other than those which may be contained in plasma or serum, hereinafter "serum"), without inoculating the environment of the culture with stromal cells and without using stromal cell conditioned media. Prior to the present invention, at least one of the foregoing agents was believed necessary in order to culture hematopoietic progenitor cells.

The growth agents of particular interest in connection with the present invention are hematopoietic growth factors. By hematopoietic growth factors, it is meant factors that influence the survival, proliferation or differentiation of hematopoietic progenitor cells. Growth agents that affect only survival and proliferation, but are not believed to promote differentiation, include the interleukins 3, 6 and 11, stem cell factor and FLT-3 ligand. Hematopoietic growth factors that promote differentiation include the colony stimulating factors such as GMCSF, GCSF, MCSF, Tpo, Epo, Oncostatin M, and interleukins other than IL-3, 6 and 11. The foregoing factors are well known to those of ordinary skill in the art. Most are commercially available. They can be obtained by purification, by recombinant methodologies or can be derived or synthesized synthetically.

"Stromal cell conditioned medium" refers to medium in which the aforementioned lymphoreticular stromal cells have been incubated. The incubation is performed for a period sufficient to allow the stromal cells to secrete factors into the medium. Such "stromal cell conditioned medium" can then be used to supplement the culture of hematopoietic progenitor cells promoting their proliferation and/or differentiation.

Thus, when cells are cultured without any of the foregoing agents, it is meant herein that the cells are cultured without the addition of such agent except as may be present in serum, ordinary nutritive media or within the blood product isolate, unfractionated or fractionated, which contains the hematopoietic progenitor cells.

According to another aspect of the invention a method for *in vivo* maintenance, expansion and/or differentiation of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells is provided. The method involves implanting into a subject a porous solid matrix having seeded hematopoietic progenitor cells, hematopoietic progenitor cell progeny, and lymphoreticular stromal cells. Implantation of matrices similar to the matrices of the invention is well known in the art (Stackpool, GJ, et al, Combined Orthopaedic Research Societies Meeting, Nov. 6-8,1995, San Diego, CA, Abstract Book p. 45; Turner, TM, et al., 21st Annual Meeting of the Society for Biomaterials, March 18-22, San Francisco, CA, Abstract Book p. 125). Such matrices are biocompatible (i.e., no immune reactivity-no rejection) and can be implanted and transplanted in a number of different tissues of a subject. Such methods are useful in a variety of ways, including the study of hematopoietic progenitor cell maintenance, expansion, differentiation and/or localization *in vivo*, in a number of different tissues of a subject, and/or between different subjects.

As used herein, a subject is a human, non-human primate, cow, horse, pig, sheep, goat, dog, cat or rodent. Human hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and human subjects are particularly important embodiments. As described above, when the matrices of the invention are used for such *in vivo* implantation studies, biological agents that promote angiogenesis (vascularization) and/or prevent/reduce inflammation may also be used for coating of the matrices. Preferred biological agents are as described above. Also as described above, the hematopoietic progenitor cells are pre-seeded onto the porous solid matrix and cultured *in vitro* according to the invention, before implantation into a subject. According to the invention, an amount of the cells is introduced *in vitro* into the porous solid matrix, and co-cultured with lymphoreticular stromal cells in an environment that is free stromal cell conditioned medium, and exogenously added hematopoietic growth factors that promote hematopoietic cell maintenance, expansion and/or differentiation, other than serum. Implantation is then carried out. In certain embodiments, stromal cell conditioned medium and exogenous hematopoietic growth factors may be added during the *in vitro* culture before implantation.

According to one aspect of the invention, a method for inducing T cell reactivity/activation, *in vitro,* is provided. Induction of T cell reactivity/activation involves co-culturing the hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and the lymphoreticular stromal cells in the presence of antigen presenting cells and an antigen, in one of the foregoing matrices, under conditions sufficient to induce the formation of T cells and/or T cell progenitors from the hematopoietic progenitor cells having specificity for the antigen. The foregoing conditions could easily be established by a person of ordinary skill in the art, without undue experimentation (see also Sprent J, et al., J Immunother, 1998, 21(3):181-187; Berridge MJ, Crit Rev Immunol, 1997, 17(2):155-178; Owen MJ, et al., Curr Opin Immunol, 1996, 8(2):191-198; Whitfield JF, et al., Mol Cell Biochem, 1979, 27(3):155-179; Fauci AS, et al., Ann Intern Med, 1983, 99(1):61-75). Antigen stimulation of T cells in the presence of APCs, induces an antigen specific response that can be measured using a proliferation assay or just by measuring IL-2 production (see discussion below). These cells can be detected by culturing T cells with antigen at an appropriate concentration (e.g., 0.1-1.0 µM tetanus toxoid) in the presence of APCs. If antigen specific T cells are present they can be detected using the assays described below under self-tolerance/anergy. Stimulation of T cells in the presence of APCs may include co-stimulation with a co-stimulatory agent. Co-stimulatory agents include lymphocyte function associated antigen-3 (LFA-3), CD2, CD40, CD80/B7-1, CD86/B7-2, OX-2, CD70, and CD82. Co-stimulatory agents may also be used in lieu of APCs, provided that MHC class II molecules and anti-CD3 antibodies are co-administered with the co-stimulatory agent(s).

One or more antigens can be used at the same time for incubation in the foregoing culture system. Preferably, the lymphoreticular stromal cells are thymic stromal cells and of murine origin when the hematopoietic progenitor cells being expanded are human. Therefore, large numbers of antigen-specific mature T and immature T cells may be obtained in a short period of time that were never before realized using existing art methodologies. The present invention thus becomes useful in a wide range of applications, including pre-exposure vaccination of individuals with *in vitro* primed T cells, treatment of cancer patients using tumor-targeted T cell immunotherapy, treatment of bone marrow transplant patients (for whom opportunistic infections, such as CMV, are problematic and yet amenable to treatment with targeted T cells such as CMV-targeted cytotoxic lymphocytes), enhancement of conventional vaccination efficacy through T cell adjuvant therapy, treatment of outbreaks of emergent or re-emergent pathogens, etc. The antigen presenting cells include cells such as dendritic cells, monocytes/macrophages, Langerhans cells, Kupfer cells, microglia, alveolar macrophages and B cells, and methods for their isolation are well known in the art. The antigen presenting cells may also be derived from hematopoietic progenitor cells *in vitro*, provided said haematopoietic progenitors are not derived from human embryos.

Immunological tolerance refers to the inhibition of a subject's ability to mount an immune response, e.g., to a donor antigen, which would otherwise occur in response to the introduction of a non-self antigen into the subject. Tolerance can involve humoral, cellular, or both humoral and cellular responses. Thymic education results in the generation of T cells capable of responding to a myriad of foreign antigens in the context of *self*-MHC, but not *self-*antigens alone. This is achieved primarily by a systematic rescue of appropriate thymocytes from programmed cell death, based on a theme of *self*-restriction, and the release of these cells into the periphery to serve as self-tolerant T cells.

Self-tolerance can be established *in vitro* under conditions known in the art that include coculturing CD34⁺ T progenitors derived from a donor (A), in the presence of thymic stroma from another individual (B). Briefly, thymic stroma is established from freshly isolated thymic tissue that is digested into a single cell suspension using a collagenase (20 µg/ml, Sigma Chemical Co.). Thymic stromal cultures are established by plating the cell suspension in 24 well plates at a concentration of 4 x 10⁶ viable cells per well in a volume of 2 ml R10 (RPMI plus 10%FCS). Cultures are incubated in a standard humidified tissue culture incubator at 37° C with 5% CO₂. After one to two days, non-adherent cells are removed by washing three times with R10. The stroma requires an additional 7-10 days to become confluent. The stroma is maintained in R10 which is changed at least twice per week. After 7-10 days in culture, CD34⁺ cells in R10 are added to the stroma at a concentration of 1-3 x 10⁵ cells per well. Cultures are fed bi-weekly using partial medium exchanges with R10 with no exogenous cytokines added to these cultures. After 14-21 days, the non-adherent cells are removed from the cultures. The remaining, attached cells are self-tolerant T cells that have developed *in vitro.*

Methods for determining if tolerance has been established *in vitro* are also known to a person of ordinary skill in the art, and involve measurement of a proliferative response to: *self* (A), as well as to the thymus donor (B), and a third party (C), peripheral blood mononuclear cells (PBMCs). Briefly, PBMCs from A, B and C are prepared by Ficoll gradient centrifugation.
1 x 10⁵ responder cells *(in vitro* generated T cells from A) are plated out in multiple replicates in a 96 well plate. Stimulator cells (PBMCs from A, B and C) are irradiated (3000 Rads) and added in 12 replicates at 1 x 10⁵ cells per well. Con-A (5 µg/ml) is used as a positive control. After 4 days 1 µCi of ³H-Thymidine is added to each well, and the plates harvested 18-24 hours later. If tolerance has been established, the *in vitro* generated T cells will respond and proliferate when mixed with an unrelated third party (C), but do not proliferate when mixed with PBMCs from self (A) or the thymic donor (B).

According to another aspect of the invention, a method for inducing T cell anergy, *in vitro,* is provided. Induction of T cell anergy involves co-culturing the hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and the lymphoreticular stromal cells in one of the foregoing matrices, in the presence of antigen under conditions sufficient to induce the formation of T cells and/or T cell progenitors and to inhibit activation of the formed T cells and/or T cell progenitors.

Anergy is defined as an unresponsive state of T cells (that is they fail to produce IL-2 on restimulation, or proliferate when restimulated) (Zamoyska R, Curr Opin Immunol, 1998, 10(1):82-87; Van Parijs L, et al., Science, 1998, 280(5361):243-248; Schwartz RH, Curr Opin Immunol, 1997, 9(3):351-357; Immunol Rev, 1993, 133:151-76). Anergy may, however, be irreversible. Anergy may be induced via antigen-specific T cell stimulation in the absence of co-stimulation (one signal vs. two signal hypothesis). Alternatively peptides of low affinity or very high concentrations of peptide even in the presence of co-stimulation can induce anergy. Anergy can be induced *in vitro* by culturing T cells in the absence of antigen presenting cells (B cells, macrophages or dendritic cells). These T cells are then exposed to antigen for example tetanus toxoid (e.g., 0.1-1.0 µM). An aliquot of the T cells is used to present antigen. This constitutes antigen presentation without co-stimulation and will induce anergy (Nelson A, et al., In Immuno, 1998, 10(9):1335-46). Alternatively T cells can be co-cultured with APCs, in the context of very high (10-100 µM) or very low (0.01-0.05 µM) tetanus toxoid, which will induce a state of unresponsiveness.

Anergy can be measured by taking the T cells described above and restimulating them with antigen (e.g., 0.1-1.0 µM tetanus toxoid) in the presence of APCs. If the cells are anergic they will not respond to antigen at an appropriate concentration in the context of APCs. Anergy is measured by culturing the cells as such for 3-5 days and measuring proliferation or the lack thereof as follows. Briefly APCs are plated out in multiple replicates in a 96 well plate, after irradiation (3000 Rads). These cells are pulsed with antigen (e.g., 0.1-1.0 µM) for 2 hours, and then T cells are added in 12 replicates at 1 x 10⁵/cells per well. Con-A (5 µg/ml) is used as a positive control. After 4 days 1 µCi of ³H-Thymidine is added to each well, and the cells are harvested 18-24 hours later. If the cells are anergic they will not proliferate in response to antigen stimulation. Alternatively, the production of IL-2 can be measured in the supernatants of the cultures described above. Supernatants are collected daily and IL-2 production is measured using a commercial ELISA assay. An additional approach includes flow cytometry based staining specific for intracellular expression of the cytokines IL-2, γIFN and TNFα using antibodies specific to the human forms of these factors (Becton Dickinson). Further, semiquantitative RT-PCR of mRNA for these factors can also be used.

According to another aspect of the invention, a method for identifying an agent suspected of affecting hematopoietic cell development is provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and an amount of lymphoreticular stromal cells into a porous, solid matrix of the invention, and co-culturing in a test co-culture the hematopoietic progenitor cells and the lymphoreticular stromal cells in the presence of at least one candidate agent suspected of affecting hematopoietic cell development. By "hematopoietic cell development" it is meant to include hematopoietic progenitor cell maintenance, expansion, differentiation, and/or cell-death apoptosis (programmed cell death). "Maintenance" includes the hematopoietic progenitor cell's ability to maintain its pluripotentiality. "Expansion" includes the hematopoietic progenitor cell's ability to divide and grow, and "differentiation" includes the hematopoietic progenitor cell's ability to differentiate toward a specific cell lineage. "Cell-death" also includes programmed cell-death (apoptosis). By "affecting" hematopoietic cell development it is therefore meant to include effects on hematopoietic progenitor cell maintenance, expansion, differentiation, and/or cell-death. Such effect (or influence) can be either positive or negative/inhibitory in nature. For example, a positive effect would be maintenance of pluripotentiality of the progenitor cells, and/or increase in the number of the pluripotential progenitor cells. A negative effect would lead into the differentiation of the progenitor cells and loss of pluripotentiality, or even progenitor cell-death. A negative effect on a particular cell population may also have a positive effect on a different cell population. For example, an inhibitory effect on a B cell lineage may result in a positive effect on, for example, a T cell lineage. The agent suspected of affecting hematopoietic cell development may be administered in the form of a transfected nucleic acid into the lymphoreticular stromal cells as well as being added straight into the media.

To determine whether the at least one candidate agent affects hematopoietic cell development in the test co-culture, the phenotype and/or genotype (as well as the numbers) of the hematopoietic cells generated in the test co-culture is compared to the phenotype and/or genotype (and numbers) of hematopoietic cells generated in a control co-culture. The control co-culture is performed under identical conditions to the test co-culture (i.e., identical initial numbers and types of both hematopoietic progenitor cells and lymphoreticular stromal cells, in an identical matrix, identical culture media, etc.), but with the exception that the at least one candidate agent suspected of affecting cell hematopoietic cell development is omitted from the control co-culture. Methods for determining the phenotype and/or genotype of hematopoietic cells are well known in the art, and a few examples can be found throughout this application.

In yet another aspect of the invention, a method for isolating from a cell culture an agent suspected of affecting hematopoietic cell development is also provided. The method involves introducing an amount of hematopoietic progenitor cells derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, and an amount of lymphoreticular stromal cells into a porous, solid matrix of the invention, co-culturing the hematopoietic progenitor cells and the lymphoreticular stromal cells and obtaining a test-supernatant (or a fraction thereof) from the co-culture. The test-supernatant (or a fraction thereof) is then compared to a control-supernatant (or a fraction thereof). By "comparing" it is meant that a profile of agents (suspected of affecting hematopoietic cell development) present in the test-supernatant and secreted from the cells of the co-culture, is compared to a similar profile of agents present in the control-supernatant and secreted from the cells of a control culture or co-culture. Methods of obtaining such profiles of secreted agents are well known in the art and include two-dimensional (2-D) gel electrophoresis. Other methods also include various types of HPLC, thin layer chromatography.

A "control culture or co-culture" may involve the culture of hematopoietic progenitor cells in a parallel culture system known in the art (e.g. U. S. Patent No. 5,677,139 by Johnson et al.), in order to obtain a result that correlates (i.e. approximates) to the result established in the co-culture system of the invention. For example, a test co-culture according to the invention that involves the co-culture of human hematopoietic progenitor cells and lymphoreticular stromal cells from a mouse thymus, gives rise to a diverse (a variety of sub-types) population of human lymphoid cells committed to the T cell lineage. The test-supernatant obtained from such co-culture is then compared to a control-supernatant obtained from a culture of human hematopoietic progenitor cells in a parallel system of the prior art (as described above) that also gives rise to a population of human lymphoid cells committed to the T cell lineage. Other examples of control cultures or co-cultures may include the co-culture of hematopoietic progenitor cells with lymphoreticular stromal cells of different tissue origin to the ones used in the test co-culture in the matrix of the invention. Additionally, the tissue may or may not be of lymphoid origin. A person of ordinary skill in the art would be able to easily choose and establish such control cultures or co-cultures. Once the profiles of agents suspected of affecting hematopoietic cell development are obtained, a subfraction of the test-supernatant that contains an agent suspected of affecting hematopoietic cell development that appears to be different or absent from the control-supernatant, can then be isolated and further characterized. For example, a candidate agent that appears to be migrating differently in a 2-D gel electrophoresis blot of the test-supernatant can be purified and further characterized using methods such as protein sequencing and mass spectrometry. Agents that appear in the 2-D gel electrophoresis blot but are absent from the blot of the test-supernatant are also suspect of affecting hematopoietic cell development and can be further purified.

The invention will be more fully understood by reference to the following examples. These examples, however, are merely intended to illustrate the embodiments of the invention and are not to be construed to limit the scope of the invention.

### Examples

### Experimental Procedures

### Isolation of Human CD34⁺ cells

Five to ten milliliters of venous umbilical cord blood (UCB) was extracted using a heparinized syringe prior to the severing of the umbilical cord during a Caeserean section delivery of a human embryo. After the umbilical cord was severed and the infant delivered, the placenta was removed by clamping the umbilical vein proximally and severing distally to the placenta. Immediately after the placenta was removed the umbilical vein was unclamped and the blood contained in the placenta drained into an appropriate heparinized container. Before processing, the cord and placenta blood was mixed together. After extraction the cord/placenta blood was diluted 2:1 with washing media (RPMI 1640, 10 IU/ml penicillin, 10 µg/ml streptomycin, 1 mM L-glutamine). The sample(s) were then underlayed with a volume of Ficoll-Hypaque (1.077 g/ml) equal to half of the diluted sample volume so that a distinct sample/Ficoll interface formed. After centrifugation for 45 minutes at 400 x g the interface containing mononuclear cells was removed. The cells were then washed by resuspending in culture medium and centrifuging for 10 minutes at 400 x g. The resulting pellet was resuspended in 6 ml of ammonium chloride lysing buffer (O.15M NH₄Cl, 1.0 mM KHCO₃, 0.1 M Na₂EDTA) for 3 minutes to lyse any remaining erythrocytes. The suspension was then diluted with media and washed twice more. After the final wash the cells were resuspended in 1-2 ml media and the number of viable cells was determined by trypan blue exclusion.

Human CD34⁺ progenitor cells were also prepared from disaggregated human fetal thymus obtained from 16-22 week old abortuses. For dissagregation procedures see below under *Mouse Thymic Stroma.*

Cells expressing the surface antigen CD34 were isolated using the Dynal CD34 Progenitor Cell Selection System (Dynal, Lake Success, N.Y.) or the MiniMACS system (Miltenyi Biotec, Bergisisch Gladbach, Germany). The mononuclear cells isolated from UCB (or bone marrow) were suspended in isolation buffer (PBS, 2% heat inactivated fetal bovine serum, 10 IU/ml penicillin, 10 p.g/ml streptomycin) at a concentration of 2.5 x 10⁷ cells/ml. The suspension was then added to magnetic anti-human CD34 beads (Dynal M-450 CD34) in a ratio of 4.0 x 10⁷ beads per ml of suspension, in a round bottom tube. (Dynabeads M-450 CD34 are superparamagnetic beads bound to monoclonal antibody specific for CD34). The mixture was vortexed gently and incubated at 4° C for 45 minutes with gentle tilt rotation using a Dynal Sample Mixer. After incubation the bead/cell mixture was resuspended in a larger volume of isolation buffer and placed in a magnetic separation device for 2 minutes to allow the cell/bead complexes to accumulate to the tube wall. While still exposed to the agent, the suspension containing the cells not bound to the magnetic beads was aspirated. The cell/bead complexes were washed three more times in this manner, pooling the suspensions containing the CD34 negative cells into the same tube. The tube containing the released cells (CD34-) was then placed on the magnetic separator to remove any remaining beads and this supernatant was transferred to a new conical tube. All CD34⁺ cells attached to beads were washed twice in a minimum of 10 ml of isolation buffer with centrifugation at 2000 rpm for 8 min. Cells bound to magnetic beads were then resuspended in 100 µl of isolation buffer per 4x10⁷ beads used, with a minimum volume of 100 µl. The CD34 positive cells were then detached from the beads by adding an equal volume of an anti-idiotype antibody (DETACHaBEAD CD34, Dynal), vortexing, and gently mixing at room temperature using a Dynal Sample Mixer for one hour. The cells were isolated from the cell/bead suspension by adding isolation buffer and placing the tube in the magnetic separation device for 2 minutes. After the beads migrated to the tube wall, the supernatant containing the CD34 positive cells was transferred to a new tube. The beads were washed three more times with the suspensions containing the released cells pooled into the same tube. The tube containing the released CD34+ cells was then placed on the magnetic separator to remove any remaining beads, and the supernatant was transferred to a new conical tube. The cells were washed twice in a minimum of 10 ml of isolation buffer with centrifugation at 2000 rpm for 10 minutes.

Alternatively, human bone marrow was obtained by posterior iliac crest aspiration from healthy adult volunteers in accordance with institutional review board guidelines and after giving informed consent. 10-15 mL of human bone marrow was collected in an heparinized sterile syringe, transported at room temperature and used within 6 hours. Bone marrow was diluted in a 5-times volume of PBS and the mononuclear cells (MNCs) separated by density gradient centrifugation over a column of Ficoll-Paque (Pharmacia Biotech Inc., Piscataway, NJ). MNCs thus obtained were washed twice in 10 mL PBS, and the remaining erythrocytes removed by lysis with ACK Lysing Buffer (Bio Whittaker, Walkersville, MD).

In order to select a more immature phenotype of progenitor cell within the CD34⁺ population, we elected to use an immunomagnetic bead selection system employing an antibody to the novel stem cell antigen, AC133. AC133 is a 5-transmembrane cell surface antigen expressed on 20-60% of human CD34⁺ cells, including the CD38^{neg/dim} subset (representing the non-lineage-committed precursors) but is not expressed on mature leukocytes (Yin AH, et al., Blood, 1997. 90:5002-12; Nfiraglia S, et al., Blood, 1997, 90: 5013-21; Buhring HJ, et al., Ann N YAcad Sci, 1999, 872: 25, discussion 38-9). Although a small number of mature CD2⁺ T-cells were transferred into our co-cultures with the AC133⁺ progenitors we do not believe that the T-cells generated in this system are derived from either CD2⁺ mature lymphocytes or CD2⁺ lymphoid-committed precursors. We, and others (Fisher AG, et al., Int Immunol, 1990, 2:571-8), have observed that the deliberate introduction of mature human T-cells into the co-cultures does not result in increased numbers of T-cells or their precursors. The AC133⁺ MNC fraction was isolated by immunomagnetic bead selection using an AC133 Cell Isolation Kit (Miltenyi Biotec Inc., Auburn, CA) according to the manufacturer's protocols.

### Mouse Thymic Stroma:

Thymi were obtained from freshly sacrificed 6 week-old B6 (BALB/C) mice. Thymi were physically disaggregated with surgical scissors in order to produce a cell suspension which also contained fragments of thymic tissue less than 0.5mm³ in size. The cell suspension containing thymic fragments was plated onto 0.5cm x 0.5cm x 0.2cm pieces of Cellfoam (80 ppi), placed in each well of a 24 well plate. Each well contained at least 5 x 10⁶ cells and 4 fragments of fetal thymus per Cellfoam block and the cells were cultured in fully supplemented IMDM. The medium in thymic cultures was changed initially at 48 hours post establishment of the culture and at three day intervals there after. On average 80% confluent thymic stromal monolayers were established on Cellfoam between 10 and 14 days. At 10 to 14 days of culture the Cellfoam blocks each containing a sub-confluent layer of thymic stroma were removed from the 24 well plate and placed in the wells of a new 24 well plate, and co-cultured with CD34⁺ cells.

### Human CD34⁺/Murine Thymic Stroma Co-Culture conditions:

Five thousand CD34⁺ cells derived from UCB or human bone marrow were then plated onto the irradiated murine thymic stroma. In the case where Cellfoam was used, CD34⁺ cells were plated directly onto the Cellfoam itself in the well of the 24 well tissue culture dish. Medium in co-cultures was changed every three days and was not supplemented with exogenous cytokines. Cells generated from the CD34⁺ cells were harvested at 7 days post establishment of the co-culture and flow-cytometric and functional studies were performed on the derived cells.

### Assessment of Immunophenotype and Function of Cells Derived from the Co-cultures:

Adherent cells were harvested with a non-trypsin isolation solution (Cell Dissociation Solution, Sigma, St. Louis, MO) to minimize alteration of surface staining characteristics. To recover adherent cells from Cellfoam, units were washed twice by immersion into PBS, saturated by brief vortexing in an excess of Cell Dissociation Solution, incubated for 20 minutes at 37°C, and centrifuged at 1500 rpm for 10 minutes.

Cells were harvested by gentle aspiration and washed twice in PBS. Harvested cells were counted and assessed for viability by trypan blue exclusion. After counting, cells were stained in a final volume of 100µL with 2% mouse serum (Dako, Carpentiera, CA) and the following fluorochrome-conjugated antibodies: TCRαβ, TCRγδ, CD2, CD3, CD4, CD8, CD14, CD33 and CD34 (Becton Dickinson, San Jose, CA). Conjugated isotype control antibodies for all four fluorochromes (FITC, PE, Peridinin chlorophyll protein (PerCP), and Allophycocyanin (APQ were used for each culture. Stained samples were washed three times with PBS, fixed with 1% paraformaldehyde, and analyzed with a FACScalibur flow cytometer (Becton Dickinson). Appropriate controls included matched isotype antibodies to establish positive and negative quadrants, as well as appropriate single color stains to establish compensation. For each sample, at least 10,000 list mode events were collected. Anti-CD3 and anti-CD14 were utilized to detect contaminating T-cells and monocytes in the CD34+ selected MC subpopulation.

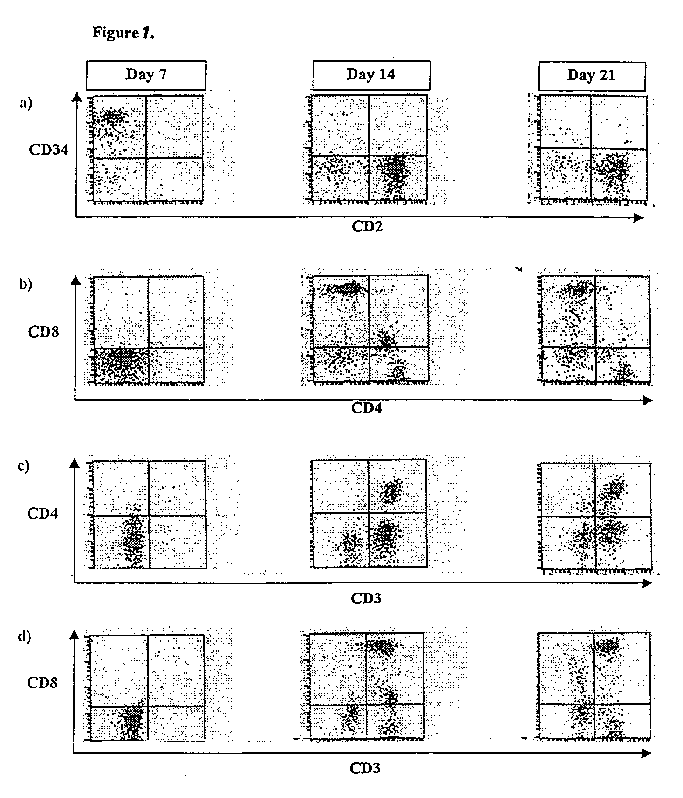

Human leukocytes were distinguishable from murine cells on immunophenotypic analysis by gating on the CD45⁺ population. After 14 days in co-culture, >70% of CD45⁺ cells coexpressed CD3, CD4, and/or CD8. It was possible to track the sequential differentiation of T-lymphoid precursors in this system over 2 weeks (Figure 1). CD34⁺ progenitors added into co-culture with a murine thymic stroma cells, on a three-dimensional matrix (Cellfoam). Non-adherent cells were harvested 7, 14 and 21 days after establishment of the co-cultures and their immunophenotype determined by FACS analysis. The data in panel (a) demonstrate the acquisition of CD2 and the down-regulation of the hematopoietic progenitor cell marker, CD34. Acquisiton of cell surface CD4 and CD8 markers occurred after 14 days in coculture; (b): discrete populations of SP CD4⁺ and SP CD8⁺ are demonstrated including their DP CD4⁺ CD8⁺ precursors. Acquisition of CD4 at day 14 was associated with acquisiotion of CD3; (c and d): all CD4⁺ cells co-expressed CD3. CD3 was co-expressed with the majority of CD8⁺ cells; those cells which were CD3⁻ CD8⁺ were found to express TCRγδ. TCRαβ was expressed by 78% of CD3⁺ cells although a smaller population (20%) of CD3+ cells expressing TCRγδ was also detectable (6% CD3⁺ CD8⁺ TCRγδ, 14% CD3⁺ CD8⁺ TCRγδ).

### T cell Function:

T cell function was assessed by determining CD69 expression in response to mitogens and ³H-Thymidine uptake in response to the mitogen Con-A. T cells generated in the co-culture system were also examined for their infectability by HIV-1 and their transducability by the MFG murine retroviral vector. T cells generated from the co-culture showed expected high levels of ³H-Thymidine uptake (10 x control unresponsive cells) in response to the mitogen ConA and a four-fold increase in the expression of the activation marker CD69 as determined by flow cytometry.

### H1V-1 Challenge of T-cells generated from HPC/thymic stromal co-cultures:

T-cells generated from HPCs were challenged with T-cell tropic isolate HIV_{IIIB} at a multiplicity of infection of 1. Titered stocks of HIV-1 were generated by standard means well known in the art. Samples of culture supernatant were removed from cultures at 3, 6, 9, 14 and 28 days post HIV challenge for HIV-1 p24 antigen estimation by ELISA (Coulter, Miami, FL). Secondly, sorted CD4⁺ T cells generated from co-cultures of BM HPCs with thymic stroma were challenged with HIV_{IIIB} at a multiplicity of infection of 1. Cell viability was also determined following challenge of monocytes and T cells with HIV-1 using trypan blue exclusion. T cells generated from HPCs were infectable with HIV-1 and produced up to 0.69ng/ml of HIV-1 p24 by day 10 of culture. Both unsorted and sorted T cells generated form the co-cultures of HPCs on murine thymic stroma on Cellfoam and exposed to heat inactivated HIV_{IIIB}, produced undetectable levels of HIV-1 p24. The viability of T cells also declined significantly following exposure to infections HIV-1. The levels of HIV-1 p24 antigen production in T cells generated form the Cellfoam co-culture system was similar to levels of HIV-1 p24 production from human activated peripheral blood T cells.

### Transduction of T cells generated from HPC/thymic stromal co-cultures with an amphotropic murine retroviral vector:

T cells generated from HPCs and expanded in IL-2 and PHA were exposed to the murine retrovirus based vector, MFG, encoding the intranuclear localized enzyme β-galactosidase at an M.O.I. of 10 on three occasions over the period of 72 hours. Titred retroviral vector was generated by standard means from a human based FLYA4 packaging cell-line. T cells were also exposed to heat inactivated MFG. Transduced cells were harvested from cultures at 7 days following retroviral exposure and stained by standard methods for the expression of the beta-galactosidase transgene. Transduction efficiencies of between 12 and 26% were observed in T cells generated from co-cultures of HPCs with murine thymic stroma grown on Cellfoam. No β-galactosidase activity was detectable in T cells exposed to the heat inactivated retroviral vector. The transduction efficiency of human T cells generated from the Cellfoam co-culture system is similar to that seen in activated peripheral blood T cells.

### mRNA extraction and cDNA synthesis:

Generated cells were also lysed and RNA was prepared from the cells for RNA PCR in order the determine T cell receptor gene expression. Messenger RNA was extracted from cells grown on a thymic monolayer. The extraction was performed using guanidinium thiocyanate and oligo-dT spun columns (QuickPrep Micro mRNA Purification Kit; Pharmacia, Piscataway, N.J.) according to the manufacturer's instructions. mRNA samples were stored at -70° C. The first strand cDNA was synthesized in a 40 µl final volume, using approximately 2 µg of mRNA, 1 µg of random primer, and 6.25 units of AMV reverse transciptase (GIBCO/BRL). Samples were incubated for 10 minutes at room temperature, 1 hour at 42° C., 5 minutes at 95° C., and 5 minutes at 4° C. RT-PCR for a number of lymphoid-specific genes (including RAG-2) was performed using reverse transcription using random primers and Moloney MuLV reverse transcriptase (GIBCO-BRL, Grand Island, NY). cDNAs were amplified using gene-specific primers, e.g., for the human RAG-2 gene which is expressed transiently only by cells undergoing lymphocyte differentiation, Vβ gene expression, and the like. PCR amplification were performed in a GeneAmp 9600 thermal cycler (Perkin Elmer, Norwalk, CT) using conditions well known in the art.

### Example 1: Viability, Immunophenotype and Function of Human Cells Generated In Co-Culture Systems

The numbers of viable cells generated in the co-culture system and their immunophenotype are shown in Table 2. Maximal human T cell proliferation was seen when human fetal thymic CD34+ cells and UCB CD34+ cells were co-cultured with murine fetal thymic stroma grown on Cellfoam. Data generated from a direct comparison of co-culture of CD34+ cells on murine thymic stroma on cell foam versus co-culture of CD34+ cells on murine stroma grown as a simple monolayer are also shown in Table 2.

T cells generated in the co-culture system were also shown to be infectable by T-tropic HIV-1_{IIIB} and these cells were also transductible at a transduction efficiency of 12-22% (n=3) with MFG vector.

### Example 2: Maintenance of Immature Progenitor Cells

According to the invention, it has also been discovered that Cellfoam cultures of thymic stromal cells are able to induce T cell differentiation of CD34⁺ progenitors and yet preserve a fraction of CD34⁺ cells. Primate CD34⁺ progenitors were cultured on either human or swine thymus that had been established on Cellfoam tissue scaffolds. After 14-21 days, CD3+CD4+CD8+ triple positive cells and CD3+CD4+ and CD3+CD8+ double positive cells are reliably recovered. In addition, the CD3- cell fraction was found to contain CD34⁺ progenitor cells after 14-21 days. These CD34⁺ cells not only were CD3-, but many were also CD2+. This demonstrates that thymus cultures in Cellfoam tissue scaffolds can support T cell differentiation while simultaneously preserving the long-lived CD34⁺ progenitor cell population. As will be evident to those skilled in the art, this surprising finding indicates that ongoing differentiation of T progeny while maintaining immature progenitor cells is possible in Cellfoam.

### Example 3: T Cell Function (Proliferation/Anergy) Assays

T cell function is evaluated by the proliferative potential to specific and non-specific antigens using standard assays. Specifically, the assay assesses the response of T cell receptor (TCR) mediated proliferation using anti-CD3 antibodies (Becton Dickinson) as well as baseline non-specific proliferation using concavalin A (Con-A). Briefly, T cells are washed and resuspended in RPMI with 10% FCS at a concentration of 10⁶ cells/ml. 100 µl (10⁵ cells) are added to each well of a 96 well plate. Cells are stimulated with either Con-A (5 µg/ml) (non-specific response) or monoclonal antibodies to CD3 in the presence of IL-2 (20 units/ml) and irradiated mononuclear cells (MCs) (10⁵ cells/well in 100 ml of RPMI with 10% FCS). Purified goat anti-mouse F(ab')₂ fragments (Kirkegard and Perry Laboratories, Gaithersberg, MD) are used as a crosslinking agent for the experimental conditions where monoclonal antibodies to CD3 are used. Wells are pretreated with 1.25 µg/ml of goat anti-mouse antibody for 45 minutes at 37°C and washed three times prior to the addition of monoclonal antibodies to CD3 and CD28. Controls included T cells alone, T cells plus irradiated MCs, and T cells plus mitogenic stimuli without IL-2 or irradiated MC. After 7 days in culture at 37°C, cell proliferation are assessed using either radio-active assays or commercially available nonradioactive, ELISA based assays (e.g. Promega). Cells are co-cultured for 5-7 days to induce proliferation of the T cells (the stimulator cells are also irradiated and thus non-proliferative). Stimulator cells alone serve as controls.

An additional approach to testing T cell function uses flow cytometry based staining for intracellular expression of the cytokines IL-2 γIFN and TNFα using antibodies specific to the human forms of these factors (Becton Dickinson). These cytokines are produced in the T progeny in the antigen specific *in vitro* proliferation assays. This allows low level detection of human cells among a high proportion of mouse cells, selectively highlighting the human progeny and excluding the mouse cells. Further, semiquantitative RT-PCR of mRNA for these factors can also be used.

In one particular example, for instance, cells removed from co-culture after 14 days showed pronounced proliferation when placed in liquid culture with complete medium and IL-2 (10 IU/mL) and phytohemagglutinin (PHA; 2 µg/mL). After a further 7 days in culture there was a 45-fold increase in cell number: >90% were CD3⁺CD4⁺ TCRαβ⁺; 3% CD3⁺CD8⁺ TCRαβ⁺ and 3% CD3⁺CD8⁺CD4⁺ TCRαβ⁺. No cells expressing TCRγδ were detected.

**TABLE 2. - USE OF THYMIC STROMA/HUMAN FETAL CD34+CELL CO-CULTURE SYSTEM**

| | Standard Protocol n=3 Human UCB CD34+ Human fetal thymic stroma | | Fetal Human Thymic stroma | 6 Week Murine Thymic stroma | Neonatal swine Thymic stroma | Fetal Human Thymic stroma | 6 Week Murine Thymic stroma | Neonatal Swine Thymic stroma |
|---|---|---|---|---|---|---|---|---|
| | | | n=3 Cell foam Thy CD34+ | n=3 Cell foam Thy CD34+ | n=3 Cell foam Thy CD34+ | n=3 Monolayer Thy CD34+ | n=3 Monolayer Thy CD34+ | n=3 Monolayer Thy CD34+ |
| | d14 | d28 | d7 | d7 | d7 | d7 | d7 | d7 |
| CD4 | 2.8 | 3.8 | 81.54 | 85.8 | 67.8 | 88.2 | 81.1 | 69.2 |
| CD3 | 1.5 | 1.1 | 12.75 | 87.1 | 80.7 | 13.1 | 90.1 | 79.2 |
| CD4/CD3 | 4.2 | 1.4 | 9.83 | 75.2 | 64.65 | 10.6 | 68.1 | 66.3 |
| CD8/CD3 | 2.1 | 2.8 | 0.13 | 74.5 | 28.8 | 0.46 | 71.9 | 28.3 |
| CD4/CD8 | 1.8 | 1 | 0.37 | 79.5 | 25.4 | 0.89 | 89.1 | 22.9 |
| CD2 | 4.1 | 12.5 | ND | 23.2 | 50.7 | ND | 20.4 | 48.1 |
| CD14 | 33.4 | 16.1 | 59.6 | 0.17 | 6.8 | 63.1 | 0.81 | 4.6 |
| CD33 | 48.2 | 20.2 | 90.61 | 0.33 | ND | 84.1 | 0.59 | ND |
| CD2/CD14 | 1.6 | 7.3 | ND | 0.85 | ND | ND | 0.26 | ND |
| CD2/CD33 | 4.8 | 12.8 | ND | 3.76 | ND | ND | 4.12 | ND |
| CD33/CD14 | | 8.4 | 60.73 | 0.13 | ND | 56.13 | 0.11 | ND |
| | | | | | | | | |
| Viable Cell Count | | | | | | | | |
| to=5,000 | 195,000 | 210,000 | 98,000 | 1,800,000 | 220,000 | 15,000 | 79,000 | 51,000 |
| | | | | | | | | |

### Example 4: T Cell Lymphopoiesis Assay

AC133⁺ progenitor cells were added to the murine thymic stromal cultures at cell densities of either 1 x 10⁵, 1 X 10⁴, or 1 x 10³ cells per well and cultured for an additional two weeks at 37°C in a 5% CO₂ humidified atmosphere. Medium in the co-cultures was changed every 4 days and was not supplemented with exogenous cytokines. Cells generated from the precursors were harvested 7 and 14 days after establishment of the co-cultures.

The selected AC133⁺ cells represented a highly purified progenitor cell population. Immunophenotypic analysis showed that >98% were CD34⁺; none co-expressed surface CD3, CD4 or CD8. A small number of contaminating CD2⁺ cells were detectable by flow cytometry within the AC133+ selected population: this comprised only 0.57% ± 0.29% (mean ± SEM; n=6) of the cells obtained from the selection process.

### Example 5: Determination of Optium Matrix Size and Input Cell Number

Having tested matrices of differing dimensions we have determined that the optimal sized matrix for use in this system measures 10mm diameter x 1 mm in depth. Similarly, input cell density appears critical for optimum T-cell generation: no lymphocytes were generated using an input cell density of less than 1 x 10⁴ cells per well. However, using 10 x 1 mm matrices and input cell densities of 1 x 10⁴ or 1 x 10⁵, we were able to generate large numbers of human cells, 71.21% ± 9.87% (mean SEM; n=7) of which were CD3⁺, after 14 days in co-culture.

### Example 6: Intra- and Inter-Sample Variability in Numbers of T-Cells Generated

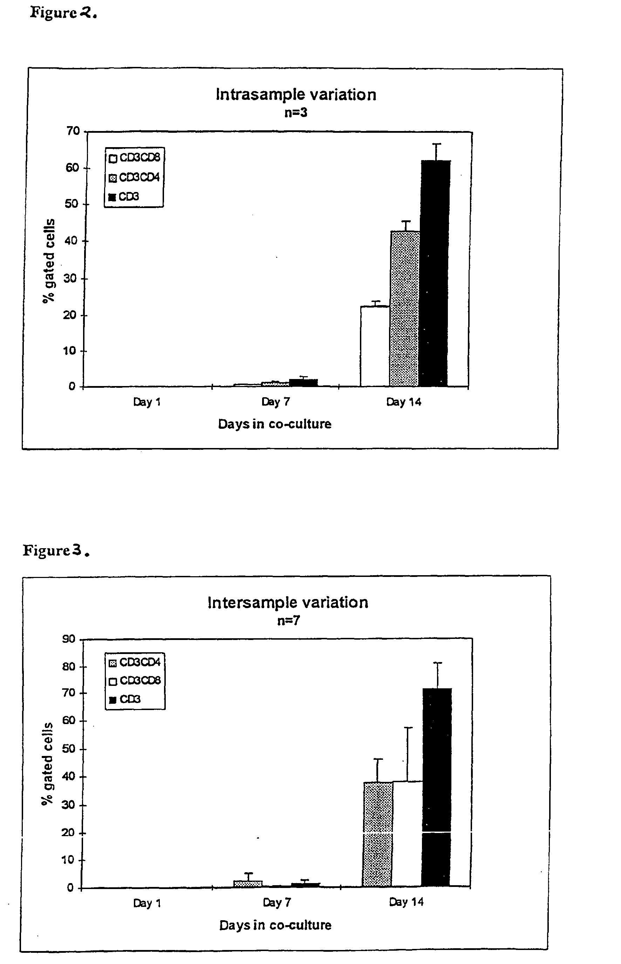

In order to determine the variation of T-cell output within the a given source of progenitors, multiple co-cultures were established using a single source of AC133⁺ cells at fixed cell densities (1 x 10⁴ cells per well) on separate 10 x 1 mm matrices of murine thymic stroma. The intrasample variation of T lymphocytes generated was analyzed by cell count using trypan blue exclusion, and by immunophenotypic analysis. Human cells were distinguished by surface expression of CD45. After 7 days in co-culture, the number of mature T-cells detected was extremely low: CD3⁺ cells represented 2.02% ± 0.87% (mean ± standard error) of the CD45⁺-gated population, CD3⁺CD4⁺ T-cells accounted for 1.0% ± 0.52% of the gated population and CD3⁺CD8⁺ 0.58% ± 0.1% of the same gated population. However, after 14 days, the numbers of T-cells were significantly higher: the proportion of CD3⁺ cells rose to 62.16% ± 4.53%; and the percentages of CD3⁺CD4⁺ and CD3⁺CD8⁺ were 42.7% ± 2.87% and 22.39% ± 1.29% respectively. These data are represented graphically in figure 2.

The intersample variation was calculated by comparing the number of T-cells generated from separate sources of CD34⁺ progenitors. In each case a fixed number of cells (1 x 10⁴ cells per well) had been introduced into co-culture. Immunophenotypic analysis of cells generated after 7 days in co-culture showed that, of the CD45 gated population, 1.57% ± 0.97% of cells expressed CD3; 2.27% ± 2.70% co-expressed CD3 and CD4; and 0.46% ± 0.23% expressed both CD3 and CD8. After 14 days, the immunophenotype of the cells harvested revealed that 71.21% ± 9.87% were CD3⁺; 37.44% ± 8.44% were CD3⁺CD4⁺, and 38.06% ± 19.13% were CD3⁺CD8⁺ as shown in figure 3.

These data demonstrate a high level of reproducibility within the system that suggests its potential in comparative analyses of input populations.

### Example 7: Analysis for TCR Excision Circles (TREC)

The TCR *Vδ* locus lies between the TCR *Vα* and TCR *J*α segments. In order to complete TCRα *VD-J* rearrangement, the TCR *Vδ* segment is excised: the 3' and 5' ends of the gene unite to form an extra-chromosomal circle of DNA termed a *TCR excision circle* (TREC) (Berenson RJ, et al., J Clin Invest, 1988, 81: 951-5; Broxmeyer HE, et al., Proc Natl Acad Sci USA, 1989, 86:3828-32). TRECs do not duplicate when the T-cell divides (Blom B., et al., J Immunol, 1997, 158:3571-7). As a consequence, TREC levels are highest in recent thymic emigrants but are sequentially diluted amongst the emigrants' progeny. TCRδ TRECs are detectable by PCR - an assay that has been shown to be a reliable tool for monitoring *de novo* T-cell generation (Tjormford GE, et al., J Exp Med, 1993, 177:1531-9). Absolute numbers of TREC positive cells will vary according to the total number of cells analyzed. We determined that the significance of TREC positivity would be most fairly interpreted by calculating the ratio of the number of TREC copies detected to the number of β-actin copies detected. We compared the level of TREC detected in T-cells harvested from the co-cultures after 14 days to TREC levels in peripheral blood mononuclear cells, B cells, AC133⁺ cells from the input population, and human fetal thymocytes. The highest TREC:bactin ratio was found in T-cells generated from the co-cultures after 14 days (0.54), followed by thymocytes from 16-22 week human fetuses (0.017). The TREC:bactin ratios from fetal and adult PBMCs and from AC133⁺ bone marrow mononuclear cells was significantly lower. These data are summarized in Table 3, below. No TREC was detected in any of the samples of B-cells tested (n=6).

**Table 3.**

| **Source** | **n** | **TREC/Bactin ratio (mean)** |
|---|---|---|
| Murine Thymocytes | 6 | 0 |
| Bone Marrow AC133+ progenitors | 3 | 0.000014 |
| Adult Peripheral blood MNCs | 3 | 0.00141 |
| Fetal Peripheral blood MNCs | 1 | 0.0024 |
| Fetal Thymocytes | 2 | 0.017 |
| T-Cells generated *in vitro* | 2 | 0.54 |

These data conclusively demonstrate that rearrangement of TCR occurs during the course of the culture period. The abundance of TREC positive cells compares favorably with that seen from fresh fetal thymus and supports the physiologic equivalence of the *in vitro* system in this aspect of T-cell differentiation.

Those skilled in the art will recognize, or be able to ascertain using no more than routine experimentation, many equivalents to the specific embodiments of the invention described herein.

## Claims

1. A method for *in vitro* production of lymphoid tissue-specific cells, comprising:
introducing hematopoietic progenitor cells and lymphoreticular stromal cells into a porous, solid matrix having interconnected pores of a pore size sufficient to permit the hematopoietic progenitor cells and the lymphoreticular stromal cells to grow throughout the matrix; and
co-culturing the hematopoietic progenitor cells and the lymphoreticular stromal cells;
wherein the lymphoreticular stromal cells are derived from at least one lymphoid soft tissue selected from the group consisting of spleen, liver, lymph node, skin, tonsil, Peyer's patches and combinations thereof, wherein the matrix has a reticulated open cell substrate comprising a thin layer of metallic material deposited onto the substrate, and wherein the amount of the lymphoreticular stromal cells introduced into the matrix is sufficient to support the growth and differentiation of the hematopoietic cells, and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

2. A method as claimed in claim 1, wherein the hematopoietic progenitor cells are genetically altered hematopoietic progenitor cells.

3. A method as claimed in claim 1, wherein the lymphoreticular stromal cells are derived from skin.

4. A method as claimed in claim 1, wherein the lymphoreticular stromal cells are genetically altered lymphoreticular stromal cells.

5. A method as claimed in claim 1, wherein the lymphoreticular stromal cells are seeded:
(a) prior to inoculating the hematopoietic progenitor cells; or
(b) at the same time as the hematopoietic progenitor cells.

6. A method as claimed in claim 1, wherein the hematopoietic progenitor cells and the lymphoreticular stromal cells are of non-embryonic human origin.

7. A method as claimed in claim 1, wherein the hematopoietic progenitor cells are of non-embryonic human origin and the lymphoreticular stromal cells are of non-human origin, preferably of murine origin.

8. A method as claimed in claims 1-7, wherein the porous solid matrix is an open cell porous matrix having a percent open space of at least 75%.

9. A method as claimed in any one of claims 1-8, wherein the porous, solid matrix having seeded hematopoietic progenitor cells and their progeny, and lymphoreticular stromal cells, is impregnated with a gelatinous agent that occupies pores of the matrix.

10. A method as claimed in claim 1, wherein the hematopoietic progenitor cells and the lymphoreticular stromal cells are cultured in an environment that is free of stromal cell conditioned medium and exogenously added hematopoetic growth factors that promote hematopoietic cell maintenance, expansion, and differentiation, other than serum, preferably wherein said growth factors are selected from the group consisting of interleukin 3, interleukin 6, interleukin 11, SCF, FLT/FLK ligand growth factors.

11. A method as claimed in claim 1, wherein the hematopoietic progenitor cells and the lymphoreticular stromal cells are cultured with an exogenously added agent selected from the group consisting of stromal cell conditioned medium, and hematopoietic growth factors that promote hematopoietic cell maintenance, expansion, differentiation, and influence cell localisation, preferably wherein said growth factors are selected from the group consisting of interleukin 3, interleukin 6, interleukin 7, interleukin 11, interleukin 12, stem cell factor, FLK-2 ligand, FLT-2 ligand, Epo, Tpo, GMCSF, GCSF, Oncostatin M, and MCSF.

12. A method as claimed in claim 1, further comprising harvesting the lymphoid tissue origin cells after the co-culturing step.

13. A composition for use in *in vivo* maintenance, expansion and/or differentiation of hematopoietic progenitor cells, comprising:
a porous, solid matrix having seeded hematopoietic progenitor cells and lymphoreticular stromal cells, the porous, solid matrix being an open cell porous matrix having a percent open space of at least 75%, prepared for implantation in a subject;
wherein the lymphoreticular stromal cells are derived from at least one lymphoid soft tissue selected from the group consisting of spleen, liver, lymph node, skin, tonsil, Peyer's patches and combinations thereof, and wherein the matrix has a reticulated open cell substrate comprising a thin layer of metallic material deposited onto the substrate, and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

14. A composition as claimed in claim 13, wherein the porous, solid matrix having seeded hematopoietic progenitor cells and lymphoreticular stromal cells is prepared for implantation by the steps of:
introducing *in vitro* hematopoietic progenitor cells and lymphoreticular stromal cells into the porous, solid matrix; and
co-culturing the hematopoietic progenitor cells and the lymphoreticular stromal cells in an environment that is free of stromal cell conditioned medium and exogenously added hematopoietic growth factors that promote hematopoietic cell maintenance, expansion and/or differentiation, other than serum.

15. A method as claimed in claim 1, or a composition as claimed in claim 14, wherein the co-culturing occurs under conditions sufficient to produce at least a 10-fold to 400-fold increase in the number of lymphoid tissue origin cells.

16. An *in vitro* method for use in inducing T cell anergy comprising:
introducing hematopoietic progenitor cells, antigen presenting cells and lymphoreticular stromal cells into a porous, solid matrix having interconnected pores of a pore size sufficient to permit the hematopoietic progenitor cells and the lymphoreticular stromal cells to grow throughout the matrix; and
co-culturing the hematopoietic progenitor cells, the antigen presenting cells and the lymphoreticular stromal cells in the presence of at least one antigen under conditions sufficient to induce the formation of T cells and/or T cell progenitors and to inhibit activation of the formed cells;
wherein the matrix has a reticulated open cell substrate comprising a thin layer of metallic material deposited onto the substrate, and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, provided the antigen presenting cells are not derived from human embryos.

17. A composition for use in inducing T cell anergy comprising:
hematopoietic progenitor cells, antigen presenting cells and lymphoreticular stromal cells provided in a porous, solid matrix having interconnected pores of a pore size sufficient to permit the hematopoietic progenitor cells and the lymphoreticular stromal cells to grow throughout the matrix;
wherein the hematopoietic progenitor cells, the antigen presenting cells and the lymphoreticular stromal cells are co-cultured in the presence of at least one antigen under conditions sufficient to induce the formation of T cells and/or T cell progenitors and to inhibit activation of the formed cells, and wherein the matrix has a reticulated open cell substrate comprising a thin layer of metallic material deposited onto the substrate, and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, provided the antigen presenting cells are not derived from human embryos.

18. An *in vitro* method for inducing T cell reactivity comprising:
introducing hematopoietic progenitor cells, antigen presenting cells and lymphoreticular stromal cells into a porous, solid matrix having interconnected pores of a pore size sufficient to permit the hematopoietic progenitor cells and the lymphoreticular stromal cells to grow throughout the matrix; and
co-culturing the hematopoietic progenitor cells, the antigen presenting cells and the lymphoreticular stromal cells in the presence of at least one antigen under conditions sufficient to induce from the hematopoietic progenitor cells the formation of T cells and/or T cell progenitors having specificity for the at least one antigen;
wherein the matrix has a reticulated open cell substrate comprising a thin layer of metallic material deposited onto the substrate, and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, provided the antigen presenting cells are not derived from human embryos.

19. A composition for use in inducing T cell reactivity comprising:
hematopoietic progenitor cells, antigen presenting cells and lymphoreticular stromal cells provided in a porous, solid matrix having interconnected pores of a pore size sufficient to permit the hematopoietic progenitor cells and the lymphoreticular stromal cells to grow throughout the matrix;
wherein the hematopoietic progenitor cells, the antigen presenting cells and the lymphoreticular stromal cells are co-cultured in the presence of at least one antigen under conditions sufficient to induce from the hematopoietic progenitor cells the formation of T cells and/or T cell progenitors having specificity for the at least one antigen, wherein the matrix has a reticulated open cell substrate comprising a thin layer of metallic material deposited onto the substrate and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells, provided the antigen presenting cells are not derived from human embryos.

20. A method as claimed in claim 18, or a composition as claimed in claim 19, wherein the antigen presenting cells are cells selected from the group consisting of dendritic cells, monocytes/macrophages, Langerhans cells, Kupfer cells, microglia, alveolar macrophages and B cells.

21. A method as claimed in claim 18, or a composition as claimed in claim 19, wherein the antigen presenting cells are derived from hematopoietic progenitor cells *in vitro.*

22. A method as claimed in claim 18, or a composition as claimed in claim 19, further comprising administering a co-stimulatory molecule to the co-culture, preferably wherein the co-stimulatory molecule is selected from the group consisting of lymphocyte function associated antigen 3 (LFA-3), CD2, CD40, CD80/B7-1, CD86/B7-2, OX-2, CD70, and CD82.

23. A composition comprising:
a porous, solid matrix having a percent open space of at least 75% and pores of a pore size sufficient to permit cells to grow throughout the matrix,
lymphoreticular stromal cells derived from at least one lymphoid soft tissue selected from the group consisting of spleen, liver, lymph node, skin, tonsil, Peyer's patches and combinations thereof, attached to the solid matrix, wherein the amount of lymphoreticular stromal cells attached to the matrix is sufficient to support the growth and differentiation of hematopoietic progenitor cells, and
hematopoietic progenitor cells attached to the matrix;
wherein the matrix has a reticulated open cell substrate comprising a thin layer of metallic material deposited onto the substrate and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

24. A composition as claimed in claim 23, wherein the hematopoietic progenitor cells are attached to the lymphoreticular stromal cells.

25. A composition as claimed in any one of claims 13-14 and 23, wherein the porous solid matrix having seeded hematopoietic progenitor cells and lymphoreticular stromal cells is impregnated with a gelatinous agent that occupies pores of the matrix.

26. A method as claimed in any one of claims 1, 16, or 18, or a composition as claimed in any one of claims 13, 17, 19 or 23, wherein the hematopoietic progenitor cells are selected from the group consisting of pluripotent stem cells, multipotent progenitor cells and progenitor cells committed to specific hematopoietic lineages.

27. A method or composition as claimed in claim 26, wherein the progenitor cells committed to specific hematopoietic lineages are committed to a lineage selected from the group consisting of T cell lineage, B cell lineage, dendritic cell lineage, Langerhans cell lineage and lymphoid tissue-specific macrophage cell lineage.

28. A method or composition as claimed in claim 26 or 27, wherein the lymphoreticular stromal cells are thymic stromal cells and the progenitor cells committed to specific hematopoietic lineages are committed to a T cell lineage.

29. A method for identifying an agent suspected of affecting hematopoietic cell development, comprising:
introducing hematopoietic progenitor cells and lymphoreticular stromal cells into a porous, solid matrix having interconnected pores of a pore size sufficient to permit the hematopoietic progenitor cells and the lymphoreticular stromal cells to grow throughout the matrix;
co-culturing in a test co-culture the hematopoietic progenitor cells and the lymphoreticular stromal cells in the presence of at least one candidate agent suspected of affecting hematopoietic cell development; and
determining whether the at least one candidate agent affects hematopoietic cell development in the test co-culture by comparing the test co-culture hematopoietic cell development to a control co-culture whereby hematopoietic progenitor cells and lymphoreticular stromal cells are co-cultured in the absence of the at least one candidate agent,
wherein the porous solid matrix is an open cell porous matrix having a percent open space of at least 75% and wherein the matrix has a reticulated open cell substrate comprising a thin layer of metallic material deposited onto the substrate and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

30. A method for isolating from a cell culture an agent suspected of affecting hematopoietic cell development, comprising:
introducing hematopoietic progenitor cells and lymphoreticular stromal cells into a porous, solid matrix having interconnected pores of a pore size sufficient to permit the hematopoietic progenitor cells and the lymphoreticular stromal cells to grow throughout the matrix;
co-culturing the hematopoietic progenitor cells and the lymyphoreticular stromal cells;
obtaining a test-supernatant from the co-culture;
comparing the test-supernatant to a control-supernatant; and
obtaining a subfraction of the test-supernatant that contains an agent suspected of affecting hematopoietic cell development that is absent from the control-supernatant,
wherein the porous, solid matrix is an open cell porous matrix having a percent open space of at least 75% and wherein the matrix has a reticulated open cell substrate comprising a thin layer of metallic material deposited onto the substrate and wherein the hematopoietic progenitor cells are derived from tissue selected from the group consisting of bone marrow, peripheral blood (including mobilized peripheral blood), umbilical cord blood, placental blood, fetal liver, non-human embryonic cells, lymphoid soft tissue and non-human aortal-gonadal-mesonephros derived cells.

31. A method as claimed in claim 30, wherein the agent:
(a) is present in the control-supernatant and absent from the test-supernatant;
(b) in the test-supernatant is different from the agent in the control-supernatant; or
(c) is further isolated by preparing a further subfraction of the test-supernatant.

32. A method as claimed in claim 30 or 31, wherein hematopoietic progenitor cell development comprises:
(a) hematopoietic progenitor cell maintenance;
(b) hematopoietic progenitor cell expansion;
(c) hematopoietic progenitor cell differentiation toward a specific cell lineage; or
(d) hematopoietic progenitor cell-death.

33. A method as claimed in claim 30 or 31, wherein the lymphoreticular stromal cells are thymic stromal cells.

34. A method or composition as claimed in any one of the preceding claims, wherein the porous solid matrix has pores defined by interconnecting ligaments having a diameter at midpoint, on average, of less than 150µm.

35. A method or composition as claimed in any one of the preceding claims, wherein the porous solid matrix is a metal-coated reticulated open cell foam of carbon containing material.

36. A method or composition as claimed in claim 34 or 35, wherein the metal is selected from the group consisting of tantalum, titanium, platinum, niobium, hafnium, tungsten, and combinations thereof, wherein said metal is coated with a biological agent selected from the group consisting of collagens, fibronectins, laminins, integrins, angiogenic factors, anti-inflammatory factors, glycosaminoglycans, vitrogen, antibodies and fragments thereof, and combinations thereof.

37. A method or composition as claimed in claim 35 or 36, wherein the metal is tantalum.

## Patentansprüche

1. Verfahren zur *in vitro*-Produktion von für lymphoides Gewebe spezifischen Zellen, umfassend:
Einführen hämatopoetischer Vorläuferzellen und lymphoretikulärer Stromazellen in eine poröse, feste Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen; und
Co-Kultivieren der hämatopoetischen Vorläuferzellen und der lymphoretikulären Stromazellen;
wobei die lymphoretikulären Stromazellen aus wenigstens einem lymphoiden Weichgewebe stammen, das aus der Gruppe ausgewählt ist, die aus Milz, Leber, Lymphknoten, Haut, Mandel, Peyer'schen Drüsen und Kombinationen davon besteht, wobei die Matrix ein netzartiges offenzelliges Substrat aufweist, das eine dünne Schicht eines auf dem Substrat abgelagerten metallischen Materials umfasst, und wobei die Menge der in die Matrix eingeführten lymphoretikulären Stromazellen ausreichend ist, um das Wachstum und die Differenzierung der hämatopoetischen Zellen zu unterstützen, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht.

2. Verfahren nach Anspruch 1, wobei die hämatopoetischen Vorläuferzellen genetisch veränderte hämatopoetische Vorläuferzellen sind.

3. Verfahren nach Anspruch 1, wobei die lymphoretikulären Stromazellen aus der Haut stammen.

4. Verfahren nach Anspruch 1, wobei die lymphoretikulären Stromazellen genetisch veränderte lymphoretikuläre Stromazellen sind.

5. Verfahren nach Anspruch 1, wobei die lymphoretikulären Stromazellen
(a) vor dem Animpfen der hämatopoetischen Vorläuferzellen; oder
(b) zur gleichen Zeit wie die hämatopoetischen Vorläuferzellen
eingesät werden.

6. Verfahren nach Anspruch 1, wobei die hämatopoetischen Vorläuferzellen und die lymphoretikulären Stromazellen nichtembryonalen menschlichen Ursprungs sind.

7. Verfahren nach Anspruch 1, wobei die hämatopoetischen Vorläuferzellen nichtembryonalen menschlichen Ursprungs und die lymphoretikulären Stromazellen nichtmenschlichen Ursprungs sind, bevorzugterweise murinen Ursprungs.

8. Verfahren nach den Ansprüchen 1 - 7, wobei die poröse feste Matrix eine offenzellige poröse Matrix ist, die einen Prozentanteil an offenem Raum von wenigstens 75 % aufweist.

9. Verfahren nach einem der Ansprüche 1-8, wobei die poröse feste Matrix, die die eingesäten hämatopoetischen Vorläuferzellen und ihre Nachkommenschaft und lymphoretikuläre Stromazellen aufweist, mit einem gelatinösen Agens imprägniert wird, das Poren der Matrix besetzt.

10. Verfahren nach Anspruch 1, wobei die hämatopoetischen Vorläuferzellen und die lymphoretikulären Stromazellen in einer Umgebung kultiviert werden, die frei von durch Stromazellen konditioniertem Medium und exogen hinzugegebenen hämatopoetischen Wachstumsfaktoren außer Serum ist, die die Erhaltung, Expansion und Differenzierung von hämatopoetischen Zellen fördern, wobei bevorzugterweise die Wachstumsfaktoren aus der Gruppe ausgewählt sind, die aus Interleukin-3, Interleukin-6, Interleukin-11, SCF, FLT/FLK-Ligand-Wachstumfaktoren besteht.

11. Verfahren nach Anspruch 1, wobei die hämatopoetischen Vorläuferzellen und die lymphoretikulären Stromazellen mit einem exogen hinzugegebenen Agens kultiviert werden, das aus der Gruppe ausgewählt ist, die aus durch Stromazellen konditioniertem Medium und hämatopoetischen Wachstumsfaktoren besteht, die die Erhaltung, Expansion und Differenzierung hämatopoetischer Wachstumsfaktoren fördern und die Zell-Lokalisierung beeinflussen, wobei bevorzugterweise die Wachstumsfaktoren aus der Gruppe ausgewählt sind, die aus Interleukin-3, Interleukin-6, Interleukin-7, Interleukin-11, Interleukin-12, Stammzellenfaktor, FLK-2-Ligand, FLT-2-Ligand, Epo, Tpo, GMCSF, GCSF, Oncostatin M und MCSF besteht.

12. Verfahren nach Anspruch 1, weiter umfassend das Ernten der Zellen mit einem Ursprung in lymphoidem Gewebe nach dem Co-Kultivierungs-Schritt.

13. Zusammensetzung zur Verwendung bei der Erhaltung, Expansion und/oder Differenzierung von hämatopoetischen Vorläuferzellen *in vivo,* umfassend:
eine poröse, feste Matrix, die eingesäte hämatopoetische Vorläuferzellen und lymphoretikuläre Stromazellen aufweist, wobei die poröse, feste Matrix eine offenzellige poröse Matrix ist, die einen Prozentanteil an offenem Raum von wenigstens 75 % aufweist und für die Implantation in einen Patienten hergerichtet ist;
wobei die lymphoretikulären Stromazellen aus wenigstens einem lymphoiden Weichgewebe stammen, das aus der Gruppe ausgewählt ist, die aus Milz, Leber, Lymphknoten, Haut, Mandel, Peyer'schen Drüsen und Kombinationen davon besteht, und wobei die Matrix ein netzartiges offenzelliges Substrat aufweist, das eine auf dem Substrat abgelagerte dünne Schicht eines metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht.

14. Zusammensetzung nach Anspruch 13, wobei die poröse, feste Matrix, die eingesäte hämatopoetische Vorläuferzellen und lymphoretikuläre Stromazellen aufweist, durch die folgenden Schritte zur Implantation hergerichtet ist:
*in vitro*-Einführen hämatopoetischer Vorläuferzellen und lymphoretikulärer Stromazellen in die poröse, feste Matrix; und
Co-Kultivieren der hämatopoetischen Vorläuferzellen und der lymphoretikulären Stromazellen in einer Umgebung, die frei von durch Stromazellen konditioniertem Medium und exogen hinzugegebenen hämatopoetischen Wachstumsfaktoren außer Serum ist, die die Erhaltung, Expansion und/oder Differenzierung von hämatopoetischen Zellen fördern.

15. Verfahren nach Anspruch 1 oder Zusammensetzung nach Anspruch 14, wobei das Co-Kultivieren unter Bedingungen stattfindet, die ausreichend sind, um wenigstens eine 10- bis 400-fache Erhöhung der Anzahl von Zellen mit Ursprung in lymphoidem Gewebe hervorzurufen.

16. *In vitro*-Verfahren zur Verwendung beim Induzieren von T-Zell-Anergie, umfassend:
Einführen hämatopoetischer Vorläuferzellen, antigenpräsentierender Zellen und lymphoretikulärer Stromazellen in eine poröse, feste Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen; und
Co-Kultivieren der hämatopoetischen Vorläuferzellen, der antigenpräsentierenden Zellen und der lymphoretikulären Stromazellen in Anwesenheit von wenigstens einem Antigen unter Bedingungen, die ausreichend sind, um die Bildung von T-Zellen und/oder T-Vorläuferzellen zu induzieren und die Aktivierung der gebildeten Zellen zu inhibieren;
wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht, vorausgesetzt, dass die antigenpräsentierenden Zellen nicht aus menschlichen Embryonen stammen.

17. Zusammensetzung zur Verwendung beim Induzieren von T-Zell-Anergie, umfassend:
hämatopoetische Vorläuferzellen, antigenpräsentierende Zellen und lymphoretikuläre Stromazellen, bereitgestellt in einer porösen, festen Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen;
wobei die hämatopoetischen Vorläuferzellen, die antigenpräsentierenden Zellen und die lymphoretikulären Stromazellen in Anwesenheit von wenigstens einem Antigen unter Bedingungen co-kultiviert werden, die ausreichend sind, um die Bildung von T-Zellen und/oder T-Vorläuferzellen zu induzieren und die Aktivierung der gebildeten Zellen zu inhibieren, und wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht, vorausgesetzt, dass die antigenpräsentierenden Zellen nicht aus menschlichen Embryonen stammen.

18. *In vitro*-Verfahren zum Induzieren von T-Zell-Reaktivität, umfassend:
Einführen von hämatopoetischen Vorläuferzellen, antigenpräsentierenden Zellen und lymphoretikulären Stromazellen in eine poröse, feste Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen; und
Co-Kultivieren der hämatopoetischen Vorläuferzellen, der antigenpräsentierenden Zellen und der lymphoretikulären Stromazellen in Gegenwart wenigstens eines Antigens unter Bedingungen, die ausreichend sind, um aus den hämatopoetischen Vorläuferzellen die Bildung von T-Zellen und/oder T-Vorläuferzellen zu induzieren, die eine Spezifität für das wenigstens eine Antigen aufweisen;
wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe abgeleitet sind, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht, vorausgesetzt, dass die antigenpräsentierenden Zellen nicht von menschlichen Embryonen stammen.

19. Zusammensetzung zur Verwendung beim Induzieren von T-Zell-Reaktivität, umfassend:
hämatopoetische Vorläuferzellen, antigenpräsentierende Zellen und lymphoretikuläre Stromazellen, bereitgestellt in einer porösen, festen Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen;
wobei die hämatopoetischen Vorläuferzellen, die antigenpräsentierenden Zellen und die lymphoretikulären Stromazellen in Anwesenheit wenigstens eines Antigens unter Bedingungen co-kultiviert werden, die ausreichend sind, um aus den hämatopoetischen Vorläuferzellen die Bildung von T-Zellen und/oder T-Vorläuferzellen zu induzieren, die eine Spezifität für das wenigstens eine Antigen aufweisen, wobei die Matrix ein netzartiges offenzelliges Substrat umfasst, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht, vorausgesetzt, dass die antigenpräsentierenden Zellen nicht aus menschlichen Embryonen stammen.

20. Verfahren nach Anspruch 18 oder Zusammensetzung nach Anspruch 19, wobei die antigenpräsentierenden Zellen Zellen sind, die aus der Gruppe ausgewählt sind, die aus dendritischen Zellen, Monozyten/Makrophagen, Langerhans'schen Zellen, Kupferzellen, Mikroglia, alveolaren Makrophagen und B-Zellen besteht.

21. Verfahren nach Anspruch 18 oder Zusammensetzung nach Anspruch 19, wobei die antigenpräsentierenden Zellen *in vitro* aus hämatopoetischen Vorläuferzellen abgeleitet sind.

22. Verfahren nach Anspruch 18 oder Zusammensetzung nach Anspruch 19, weiter umfassend das Verabreichen eines co-stimulierenden Moleküls an die Co-Kultur, wobei das co-stimulierende Molekül bevorzugterweise aus der Gruppe ausgewählt ist, die aus Lymphozyten-Funktion-assoziiertem Antigen 3 (LFA-3), CD2, CD40, CD80/B7-1, CD86/B7-2, OX-2, CD70 und CD82 besteht.

23. Zusammensetzung, umfassend:
eine poröse, feste Matrix, die einen Prozentanteil an offenem Raum von mindestens 75 % und Poren einer Porengröße aufweist, die ausreichend ist, um Zellen zu erlauben, überall in der Matrix zu wachsen,
lymphoretikuläre Stromazellen, die aus wenigstens einem lymphoiden Weichgewebe stammen, das aus der Gruppe ausgewählt ist, die aus Milz, Leber, Lymphknoten, Haut, Mandel, Peyer'schen Drüsen und Kombinationen davon besteht, die an der festen Matrix befestigt sind, wobei die Menge der an der Matrix befestigten lymphoretikulären Stromazellen ausreichend ist, um das Wachstum und die Differenzierung von hämatopoetischen Vorläuferzellen zu unterstützen, und
hämatopoetische Vorläuferzellen, die an der Matrix befestigt sind,
wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht.

24. Zusammensetzung nach Anspruch 23, wobei die hämatopoetischen Vorläuferzellen an den lymphoretikulären Stromazellen befestigt sind.

25. Zusammensetzung nach einem jeden der Ansprüche 13 - 14 und 23, wobei die poröse feste Matrix, die eingesäte hämatopoetische Vorläuferzellen und lymphoretikuläre Stromazellen aufweist, mit einem gelatinösen Agens imprägniert ist, das Poren der Matrix besetzt.

26. Verfahren nach einem jeden der Ansprüche 1, 16 oder 18 oder eine Zusammensetzung nach einem jeden der Ansprüche 13, 17, 19 oder 23, wobei die hämatopoetischen Vorläuferzellen aus der Gruppe ausgewählt sind, die aus pluripotenten Stammzellen, multipotenten Vorläuferzellen und Vorläuferzellen besteht, die auf spezifische hämatopoetische Linien festgelegt sind.

27. Verfahren oder Zusammensetzung nach Anspruch 26, wobei die Vorläuferzellen, die auf spezifische hämatopoetische Linien festgelegt sind, auf eine Linie festgelegt sind, die aus der Gruppe ausgewählt ist, die aus der T-Zell-Linie, B-Zell-Linie, der Linie dendritischer Zellen, der Linie Langerhans'scher Zellen und der Zell-Linie für für lymphoides Gewebe spezifischen Makrophagen besteht.

28. Verfahren oder Zusammensetzung nach Anspruch 26 oder 27, wobei die lymphoretikulären Stromazellen Thymusstromazellen sind und die Vorläuferzellen, die auf spezifische hämatopoetische Linien festgelegt sind, auf eine T-Zell-Linie festgelegt sind.

29. Verfahren zum Identifizieren eines Agens, von dem vermutet wird, dass es die Entwicklung hämatopoetischer Zellen beeinflusst, umfassend:
Einführen hämatopoetischer Vorläuferzellen und lymphoretikulärer Stromazellen in eine poröse, feste Matrix, die miteinander verbundene Poren einer Porengröße aufweist, die ausreichend ist, um die hämatopoetischen Vorläuferzellen und die lymphoretikulären Stromazellen überall in der Matrix wachsen zu lassen;
Co-Kultivieren der hämatopoetischen Vorläuferzellen und der lymphoretikulären Stromazellen in einer Test-Co-Kultur in Anwesenheit von wenigstens einem Kandidaten-Agens, von dem vermutet wird, dass es die Entwicklung hämatopoetischer Zellen beeinflusst; und
Bestimmen, ob das wenigstens eine Kandidatenagens die Entwicklung hämatopoetischer Zellen in der Test-Co-Kultur beeinflusst, durch Vergleichen der Entwicklung hämatopoetischer Zellen in der Test-Co-Kultur mit einer Kontroll-Co-Kultur, wobei hämatopoetische Vorläuferzellen und lymphoretikuläre Stromazellen in Abwesenheit des wenigstens einen Kandidaten-Agens co-kultiviert werden,
wobei die poröse, feste Matrix eine offenzellige poröse Matrix ist, die einen Prozentanteil an offenem Raum von wenigstens 75 % aufweist, und wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht.

30. Verfahren zum Isolieren eines Agens aus einer Zellkultur, von dem vermutet wird, dass es die Entwicklung hämatopoetischer Zellen beeinflusst, umfassend:
Einführen hämatopoetischer Vorläuferzellen und lymphoretikulärer Stromazellen in eine poröse, feste Matrix, die miteinander verbundene Poren einer Porengröße aufweist, die ausreichend ist, um den hämatopoetischen Vorläuferzellen und den lymphoretikulären Stromazellen zu erlauben, überall in der Matrix zu wachsen;
Co-Kultivieren der hämatopoetischen Vorläuferzellen und der lymphoretikulären Stromazellen;
Erhalten eines Test-Überstandes aus der Co-Kultur;
Vergleichen des Test-Überstandes mit einem Kontroll-Überstand; und
Erhalten einer Subfraktion des Test-Überstands, die ein Agens enthält, von dem vermutet wird, dass es die Entwicklung hämatopoetischer Zellen beeinflusst, und das im Kontroll-Überstand nicht vorhanden ist,
wobei die poröse, feste Matrix eine offenzellige poröse Matrix ist, die einen Prozentanteil an offenem Raum von wenigstens 75 % aufweist, und wobei die Matrix ein netzartiges offenzelliges Substrat aufweist, das eine dünne Schicht eines auf dem Substrat abgelagerten metallischen Materials umfasst, und wobei die hämatopoetischen Vorläuferzellen aus Gewebe stammen, das aus der Gruppe ausgewählt ist, die aus Knochenmark, peripherem Blut (einschließlich mobilisiertem peripherem Blut), Nabelschnurblut, plazentalem Blut, fötaler Leber, nichtmenschlichen embryonalen Zellen, lymphoidem Weichgewebe und von nichtmenschlichem aortal-gonadalem Mesonephros stammenden Zellen besteht.

31. Verfahren nach Anspruch 30, wobei das Agens:
(a) im Kontroll-Überstand anwesend und im Test-Überstand abwesend ist;
(b) sich im Test-Überstand von dem Agens im Kontroll-Überstand unterscheidet; oder
(c) durch das Herstellen einer weiteren Subfraktion des Test-Überstandes weiter isoliert wird.

32. Verfahren nach Anspruch 30 oder 31, wobei die Entwicklung hämatopoetischer Vorläuferzellen:
(a) die Erhaltung hämatopoetischer Vorläuferzellen;
(b) die Expansion hämatopoetischer Vorläuferzellen;
(c) die Differenzierung hämatopoetischer Vorläuferzellen hin zu einer spezifischen Zell-Linie; oder
(d) den Tod hämatopoetischer Vorläuferzellen
umfasst.

33. Verfahren nach Anspruch 30 oder 31, wobei die lymphoretikulären Stromazellen Thymusstromazellen sind.

34. Verfahren oder Zusammensetzung nach einem der vorangehenden Ansprüche, wobei die poröse feste Matrix Poren aufweist, die durch miteinander verbundene Ligamente definiert sind, die am Mittelpunkt im Durchschnitt einen Durchmesser von weniger als 150 µm aufweisen.

35. Verfahren oder Zusammensetzung nach einem der vorangehenden Ansprüche, wobei die poröse feste Matrix ein metallbeschichteter netzartiger offenzelliger Schaum aus einem Kohlenstoff enthaltenden Material ist.

36. Verfahren oder Zusammensetzung nach Anspruch 34 oder 35, wobei das Metal aus der Gruppe ausgewählt ist, die aus Tantal, Titan, Platin, Niob, Hafnium, Wolfram oder Kombinationen daraus besteht, wobei das Metal mit einem biologischen Agens beschichtet ist, das aus der Gruppe ausgewählt ist, die aus Kollagenen, Fibronektinen, Lamininen, Integrinen, angiogenen Faktoren, anti-entzündlichen-Faktoren, Glycosaminoglycanen, Vitrogen, Antikörpern und Fragmenten davon, und Kombinationen davon besteht.

37. Verfahren oder Zusammensetzung nach Anspruch 35 oder 36, wobei das Metall Tantal ist.

## Revendications

1. Procédé de production *in vitro* de cellules spécifiques d'un tissu lymphoïde consistant à :
introduire des cellules progénitrices hématopoïétiques et des cellules stromales lymphoréticulaires dans une matrice solide poreuse comportant des pores interconnectés ayant une taille de pore suffisante pour permettre aux cellules progénitrices hématopoïétiques et aux cellules stromales lymphoréticulaires de croître dans la matrice ; et
co-cultiver les cellules progénitrices hématopoïétiques et les cellules stromales lymphoréticulaires ;
où les cellules stromales lymphoréticulaires sont dérivées d'au moins un tissu mou lymphoïde sélectionné dans le groupe consistant en la rate, le foie, un ganglion lymphatique, la peau, l'amygdale, les plaques de Peyer et des combinaisons de ceux-ci, où la matrice possède un substrat à cellules ouvertes réticulé comprenant une fine couche de matériau métallique déposée sur le substrat, et où la quantité de cellules stromales lymphoréticulaires introduite dans la matrice est suffisante pour étayer la croissance et la différenciation des cellules hématopoïétiques, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros.

2. Procédé selon la revendication 1, dans lequel les cellules progénitrices hématopoïétiques sont des cellules progénitrices hématopoïétiques génétiquement altérées.

3. Procédé selon la revendication 1, dans lequel les cellules stromales lymphoréticulaires sont dérivées de la peau.

4. Procédé selon la revendication 1, dans lequel les cellules stromales lymphoréticulaires sont des cellules stromales lymphoréticulaires génétiquement altérées.

5. Procédé selon la revendication 1, dans lequel les cellules stromales lymphoréticulaires sont ensemencées :
(a) avant d'inoculer les cellules progénitrices hématopoïétiques ; ou
(b) en même temps que les cellules progénitrices hématopoïétiques.

6. Procédé selon la revendication 1, dans lequel les cellules progénitrices hématopoïétiques et les cellules stromales lymphoréticulaires sont d'origine humaine non embryonnaire.

7. Procédé selon la revendication 1, dans lequel les cellules progénitrices hématopoïétiques sont d'origine humaine non embryonnaire et les cellules stromales lymphoréticulaires sont d'origine non humaine, de préférence d'origine murine.

8. Procédé selon la revendication 1 à 7, dans lequel la matrice solide poreuse est une matrice poreuse à cellules ouvertes comportant un pourcentage d'espace ouvert d'au moins 75 %.

9. Procédé selon l'une quelconque des revendications 1 à 8, dans lequel la matrice solide poreuse où sont ensemencées des cellules progénitrices hématopoïétiques et leur progénie, ainsi que des cellules lymphoréticulaires, est imprégnée d'un agent gélatineux qui occupe les pores de la matrice.

10. Procédé selon la revendication 1, dans lequel les cellules progénitrices hématopoïétiques et les cellules stromales lymphoréticulaires sont cultivées dans un environnement qui est exempt de milieu conditionné en cellules stromales et de facteurs de croissance hématopoïétiques ajoutés de manière exogène qui favorisent la maintenance, l'expansion et la différenciation des cellules hématopoïétiques, autres que du sérum, où de préférence lesdits facteurs de croissance sont sélectionnés dans le groupe consistant en les facteurs de croissance interleukine 3, interleukine 6, interleukine 11, SCF, ligand FLT/FLK.

11. Procédé selon la revendication 1, dans lequel les cellules progénitrices hématopoïétiques et les cellules stromales lymphoréticulaires sont cultivées avec un agent ajouté de manière exogène qui est sélectionné dans le groupe consistant en un milieu conditionné en cellules stromales, et des facteurs de croissance hématopoïétiques qui favorisent la maintenance, l'expansion, la différenciation des cellules hématopoïétiques, et influencent la localisation cellulaire, où de préférence lesdits facteurs de croissance sont sélectionnés dans le groupe consistant en l'interleukine 3, l'interleukine 6, l'interleukine 7, l'interleukine 11, l'interleukine 12, le facteur des cellules souches, le ligand FLK-2, le ligand FLT-2, l'Epo, la Tpo, le GMCSF, le GCSF, l'oncostatine M, et le MCSF.

12. Procédé selon la revendication 1, qui consiste en outre à récolter les cellules originaires d'un tissu lymphoïde après l'étape de co-culture.

13. Composition destinée à être utilisée dans la maintenance, l'expansion et/ou la différenciation *in vivo* de cellules progénitrices hématopoïétiques, comprenant :
une matrice solide poreuse où sont ensemencées des cellules progénitrices hématopoïétiques et des cellules stromales lymphoréticulaires, la matrice solide poreuse étant une matrice poreuse à cellules ouvertes qui comporte un pourcentage d'espace ouvert d'au moins 75 %, préparée pour une implantation chez un sujet ;
où les cellules stromales lymphoréticulaires sont dérivées d'au moins un tissu mou lymphoïde sélectionné dans le groupe consistant en la rate, le foie, un ganglion lymphatique, la peau, l'amygdale, les plaques de Peyer et des combinaisons de ceux-ci, et où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros.

14. Composition selon la revendication 13, dans laquelle la matrice solide poreuse où sont ensemencées des cellules progénitrices hématopoïétiques et des cellules stromales lymphoréticulaires est préparée pour une implantation par les étapes consistant à :
introduire des cellules progénitrices hématopoïétiques et des cellules stromales lymphoréticulaires *in vitro* dans la matrice solide poreuse ; et
co-cultiver les cellules progénitrices hématopoïétiques et les cellules stromales lymphoréticulaires dans un environnement qui est exempt de milieu conditionné en cellules stromales et de facteurs de croissance hématopoïétiques ajoutés de manière exogène qui favorisent la maintenance, l'expansion et/ou la différenciation de cellules hématopoïétiques, autres que du sérum.

15. Procédé selon la revendication 1, ou composition selon la revendication 14, où la co-culture est réalisée dans des conditions suffisantes pour produire une augmentation du nombre de cellules originaires d'un tissu lymphoïde d'au moins 10 fois à 400 fois.

16. Procédé *in vitro* destiné à être utilisé pour induire une anergie de lymphocytes T, consistant à :
introduire des cellules progénitrices hématopoïétiques, des cellules présentatrices d'antigène et des cellules stromales lymphoréticulaires dans une matrice solide poreuse comportant des pores interconnectés ayant une taille de pore suffisante pour permette aux cellules progénitrices hématopoïétiques et aux cellules stromales lymphoréticulaires de croître dans la matrice ; et
co-cultiver les cellules progénitrices hématopoïétiques, les cellules présentatrices d'antigène et les cellules stromales lymphoréticulaires en présence d'au moins un antigène dans des conditions suffisantes pour induire la formation de lymphocytes T et/ou de progéniteurs de lymphocytes T et pour inhiber l'activation des cellules formées ;
où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros, à condition que les cellules présentatrices d'antigène ne soient pas dérivées d'embryons humains.

17. Composition destinée à être utilisée pour induire une anergie de lymphocytes T comprenant :
des cellules progénitrices hématopoïétiques, des cellules présentatrices d'antigène et des cellules stromales lymphoréticulaires mises à disposition dans une matrice solide poreuse comportant des pores interconnectés ayant une taille de pore suffisante pour permette aux cellules progénitrices hématopoïétiques et aux cellules stromales lymphoréticulaires de croître dans la matrice ;
où les cellules progénitrices hématopoïétiques, les cellules présentatrices d'antigène et les cellules stromales lymphoréticulaires sont co-cultivées en présence d'au moins un antigène dans des conditions suffisantes pour induire la formation de lymphocytes T et/ou de progéniteurs de lymphocytes T et pour inhiber l'activation des cellules formées, et où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros, à condition que les cellules présentatrices d'antigène ne soient pas dérivées d'embryons humains.

18. Procédé *in vitro* pour induire une réactivité de lymphocytes T consistant à :
introduire des cellules progénitrices hématopoïétiques, des cellules présentatrices d'antigène et des cellules stromales lymphoréticulaires dans une matrice solide poreuse comportant des pores interconnectés ayant une taille de pore suffisante pour permettre aux cellules progénitrices hématopoïétiques et aux cellules stromales lymphoréticulaires de croître dans la matrice ; et
co-cultiver les cellules progénitrices hématopoïétiques, les cellules présentatrices d'antigène et les cellules stromales lymphoréticulaires en présence d'au moins un antigène dans des conditions suffisantes pour induire, à partir des cellules progénitrices hématopoïétiques, la formation de lymphocytes T et/ou de progéniteurs de lymphocytes T ayant une spécificité pour ledit au moins un antigène ;
où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros, à condition que les cellules présentatrices d'antigène ne soient pas dérivées d'embryons humains.

19. Composition destinée à être utilisée pour induire une réactivité de lymphocytes T comprenant :
des cellules progénitrices hématopoïétiques, des cellules présentatrices d'antigène et des cellules stromales lymphoréticulaires mises à disposition dans une matrice solide poreuse comportant des pores interconnectés ayant une taille de pore suffisante pour permette aux cellules progénitrices hématopoïétiques et aux cellules stromales lymphoréticulaires de croître dans la matrice ;
où les cellules progénitrices hématopoïétiques, les cellules présentatrices d'antigène et les cellules stromales lymphoréticulaires sont co-cultivées en présence d'au moins un antigène dans des conditions suffisantes pour induire, à partir des cellules progénitrices hématopoïétiques, la formation de lymphocytes T et/ou de progéniteurs de lymphocytes T ayant une spécificité pour ledit au moins un antigène, et où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros, à condition que les cellules présentatrices d'antigène ne soient pas dérivées d'embryons humains.

20. Procédé selon la revendication 18, ou composition selon la revendication 19, où les cellules présentatrices d'antigène sont des cellules sélectionnées dans le groupe consistant en des cellules dendritiques, des monocytes/macrophages, des cellules de Langerhans, des cellules de Kupffer, la microglie, des macrophages alvéolaires et des lymphocytes B.

21. Procédé selon la revendication 18, ou composition selon la revendication 19, où les cellules présentatrices d'antigène sont dérivées de cellules progénitrices hématopoïétiques *in vitro.*

22. Procédé selon la revendication 18, ou composition selon la revendication 19, consistant en outre à administrer une molécule costimulatrice à la co-culture, où de préférence la molécule costimulatrice est sélectionnée dans le groupe consistant en l'antigène 3 associé à la fonction lymphocytaire (LFA-3), CD2, CD40, CD80/B7-1, CD86/B7-2, OX-2, CD70, et CD82.

23. Composition comprenant :
une matrice solide poreuse comportant un pourcentage d'espace ouvert d'au moins 75 % et des pores ayant une taille de pore suffisante pour permettre à des cellules de croître dans la matrice,
des cellules stromales lymphoréticulaires dérivées d'au moins un tissu mou lymphoïde sélectionné dans le groupe consistant en la rate, le foie, un ganglion lymphatique, la peau, l'amygdale, les plaques de Peyer et des combinaisons de ceux-ci, qui est attaché à la matrice solide, et où la quantité de cellules stromales lymphoréticulaires attachées à la matrice est suffisante pour étayer la croissance et la différenciation de cellules progénitrices hématopoïétiques, et
des cellules progénitrices hématopoïétiques attachées à la matrice ;
où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros.

24. Composition selon la revendication 23, où les cellules progénitrices hématopoïétiques sont attachées aux cellules stromales lymphoréticulaires.

25. Composition selon l'une quelconque des revendications 13 à 14 et 23, où la matrice solide poreuse où sont ensemencées des cellules progénitrices hématopoïétiques et des cellules stromales lymphoréticulaires est imprégnée d'un agent gélatineux qui occupe les pores de la matrice.

26. Procédé selon l'une quelconque des revendications 1, 16, ou 18, ou composition selon l'une quelconque des revendications 13, 17, 19 ou 23, où les cellules progénitrices hématopoïétiques sont sélectionnées dans le groupe consistant en des cellules souches pluripotentes, des cellules progénitrices multipotentes et des cellules progénitrices déterminées en des lignées hématopoïétiques spécifiques.

27. Procédé ou composition selon la revendication 26, où les cellules progénitrices déterminées en lignées hématopoïétiques spécifiques sont déterminées en une lignée sélectionnée dans le groupe consistant en une lignée de lymphocyte T, une lignée de lymphocyte B, une lignée de cellule dendritique, une lignée de cellule de Langerhans et une lignée de cellule macrophage spécifique d'un tissu lymphoïde.

28. Procédé ou composition selon la revendication 26 ou 27, où les cellules stromales lymphoréticulaires sont des cellules stromales thymiques et les cellules progénitrices déterminées en lignées hématopoïétiques spécifiques sont déterminées en une lignée de lymphocyte T.

29. Procédé pour identifier un agent suspecté d'affecter un développement de cellules hématopoïétiques, consistant à :
introduire des cellules progénitrices hématopoïétiques et des cellules stromales lymphoréticulaires dans une matrice solide poreuse comportant des pores interconnectés ayant une taille de pore suffisante pour permettre aux cellules progénitrices hématopoïétiques et aux cellules stromales lymphoréticulaires de croître dans la matrice ;
co-cultiver dans une co-culture de test les cellules progénitrices hématopoïétiques et les cellules stromales lymphoréticulaires en présence d'au moins un agent candidat suspecté d'affecter le développement de cellules hématopoïétiques ; et
déterminer si ledit au moins un agent candidat affecte le développement des cellules hématopoïétiques dans la co-culture de test en comparant le développement des cellules hématopoïétiques de la co-culture de test à une co-culture de contrôle moyennant quoi les cellules progénitrices et les cellules stromales lymphoréticulaires sont co-cultivées en l'absence dudit au moins un agent candidat,
où la matrice solide poreuse est une matrice poreuse à cellules ouvertes ayant un pourcentage d'espace ouvert d'au moins 75 % et où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros.

30. Procédé pour isoler à partir d'une culture cellulaire un agent suspecté d'affecter le développement de cellules hématopoïétiques, consistant à :
introduire des cellules progénitrices hématopoïétiques et des cellules stromales lymphoréticulaires dans une matrice solide poreuse comportant des pores interconnectés ayant une taille de pore suffisante pour permettre aux cellules progénitrices hématopoïétiques et aux cellules stromales lymphoréticulaires de croître dans la matrice ;
co-cultiver les cellules progénitrices hématopoïétiques et les cellules stromales lymphoréticulaires ;
obtenir un surnageant de test à partir de la co-culture ;
comparer le surnageant de test à un surnageant de contrôle ; et
obtenir une sous-fraction du surnageant de test qui contient un agent suspecté d'affecter le développement des cellules hématopoïétiques qui est absent du surnageant de contrôle,
où la matrice solide poreuse est une matrice poreuse à cellules ouvertes ayant un pourcentage d'espace ouvert d'au moins 75 % et où la matrice comporte un substrat à cellules ouvertes réticulé comprenant une fine couche de matériau métallique déposée sur le substrat, et où les cellules progénitrices hématopoïétiques sont dérivées d'un tissu sélectionné dans le groupe consistant en la moelle osseuse, le sang périphérique (y compris du sang périphérique mobilisé), le sang du cordon ombilical, le sang placentaire, le foie foetal, des cellules embryonnaires non humaines, un tissu mou lymphoïde et des cellules non humaines dérivées de la région aorte-gonades-mésonéphros.

31. Procédé selon la revendication 30, où l'agent :
(a) est présent dans le surnageant de contrôle et est absent du surnageant de test ;
(b) dans le surnageant de test est différent de l'agent dans le surnageant de contrôle ; ou
(c) est en outre isolé en préparant une autre sous-fraction du surnageant de test.

32. Procédé selon la revendication 30 ou 31, dans lequel le développement des cellules progénitrices hématopoïétiques comprend :
(a) une maintenance des cellules progénitrices hématopoïétiques ;
(b) une expansion des cellules progénitrices hématopoïétiques ;
(c) une différenciation des cellules progénitrices hématopoïétiques en une lignée cellulaire spécifique ; ou
(d) une mort des cellules progénitrices hématopoïétiques.

33. Procédé selon la revendication 30 ou 31, dans lequel les cellules stromales lymphoréticulaires sont des cellules stromales thymiques.

34. Procédé ou composition selon l'une quelconque des revendications précédentes, où la matrice solide poreuse comporte des pores définis par des ligaments d'interconnexion ayant un diamètre médian, en moyenne, inférieur à 150 µm.

35. Procédé ou composition selon l'une quelconque des revendications précédentes, où la matrice solide poreuse est un matériau contenant une mousse de carbone à cellules ouvertes réticulée enrobée de métal.

36. Procédé ou composition selon la revendication 34 ou 35, où le métal est sélectionné dans le groupe consistant en le tantale, le titane, le platine, le niobium, le hafnium, le tungstène, et des combinaisons de ceux-ci, où ledit métal est enrobé avec un agent biologique sélectionné dans le groupe consistant en des collagènes, des fibronectines, des laminines, des intégrines, des facteurs angiogènes, des facteurs anti-inflammatoires, des glycosaminoglycanes, du vitrogène, des anticorps et des fragments de ceux-ci, et des combinaisons de ceux-ci.

37. Procédé ou composition selon la revendication 35 ou 36, où le métal est le tantale.
